# EUROPEAN PATENT SPECIFICATION

(11) **EP 2 703 495 B1**
(45) Date of publication and mention of the grant of the patent: **09.05.2018**
(21) Application number: 12765602.3
(22) Date of filing: 29.03.2012
(51) Int. Cl.: C12Q 1/04, A61B 10/00, A61K 49/00, G01N 21/64, G01N 33/48, G01N 33/52

(54) **METHOD FOR DETECTING CANCER CELL USING FLUORESCENTLY LABELED L-GLUCOSE DERIVATIVE, AND CANCER CELL-IMAGING AGENT COMPRISING FLUORESCENTLY LABELED L-GLUCOSE DERIVATIVE**
VERFAHREN ZUM NACHWEIS VON KREBSZELLEN UNTER VERWENDUNG EINES FLUORESZENZMARKIERTEN L-GLUCOSE-DERIVATS UND KREBSZELLEN-BILDGEBUNGSMITTEL MIT DEM FLUORESZENZMARKIERTEN L-GLUCOSE-DERIVAT
PROCÉDÉ POUR DÉTECTER UNE CELLULE CANCÉREUSE EN UTILISANT UN DÉRIVÉ DE L-GLUCOSE MARQUÉ PAR FLUORESCENCE, ET AGENT D'IMAGERIE DE CELLULES CANCÉREUSES COMPRENANT UN DÉRIVÉ DE L-GLUCOSE MARQUÉ PAR FLUORESCENCE

(30) Priority: 31.03.2011 JP 2011079418
(43) Date of publication of application: 05.03.2014
(73) Proprietor: Hirosaki University, Hirosaki-shi, Aomori 036-8560 (JP); Riken, Wako-shi, Saitama 351-0198 (JP); Peptide Institute, Inc., Ibaraki-shi, Osaka 567-0085 (JP)
(72) Inventor: YAMADA, Katsuya, Hirosaki-shi Aomori 036-8560 (JP); ONOE, Hirotaka, Kobe-shi Hyogo 650-0047 (JP); TESHIMA, Tadashi, Osaka 567-0085 (JP); YAMAMOTO, Toshihiro, Osaka 567-0085 (JP)
(74) Representative: Maiwald Patentanwalts GmbH
(86) International application number: PCT/JP2012/058439
(87) International publication number: WO 2012/133688

(56) References cited:
- WO-A1-2010/016587
- WO-A1-2010/016587
- WO-A2-03/059149
- US-A1- 2009 317 829
- US-A1- 2009 317 829
- H. JADVAR ET AL: "18F-FDG Uptake in Lung, Breast, and Colon Cancers: Molecular Biology Correlates and Disease Characterization", THE JOURNAL OF NUCLEAR MEDICINE, vol. 50, no. 11, 16 October 2009 (2009-10-16), pages 1820-1827, XP055144574, ISSN: 0161-5505, DOI: 10.2967/jnumed.108.054098
- TOSHIHIRO YAMAMOTO ET AL: "Synthesis of 2-NBDLG, a fluorescent derivative of l-glucosamine; the antipode of d-glucose tracer 2-NBDG", TETRAHEDRON LETTERS, vol. 49, no. 48, 24 November 2008 (2008-11-24), pages 6876-6878, XP055002356, ISSN: 0040-4039, DOI: 10.1016/j.tetlet.2008.09.089
- TOSHIHIRO YAMAMOTO ET AL: "Syntheses of 2-NBDG analogues for monitoring stereoselective uptake of d-glucose", BIOORGANIC & MEDICINAL CHEMISTRY LETTERS, vol. 21, no. 13, 10 May 2011 (2011-05-10), pages 4088-4096, XP055144449, ISSN: 0960-894X, DOI: 10.1016/j.bmcl.2011.04.148
- YAMAMOTO TOSHIHIRO ET AL.: 'Synthesis of 2-NBDLG, a fluorescent derivative of 1-glucosamine; the antipode of d-glucose tracer 2-NBDG.' TETRAHEDRON LETTERS vol. 49, no. 48, 2008, pages 6876 - 6878, XP025533080

## Description

### TECHNICAL FIELD

The present invention relates to a method for detecting cancer cells *in vitro* using a fluorescently labeled L-glucose derivative. Further, the present invention relates to a fluorescently labeled L-glucose derivative for use in an *in vivo* diagnosing method for detecting cancer cells.

### BACKGROUND ART

Detecting a cancer with a high accuracy is important for finding and therapy thereof. In general, cancer tissue is not an aggregate of cells showing a uniform nature, but an aggregate of cells showing various morphological or functional features and degrees of differentiation. There is also a case in which cancer tissue is scattered in normal cells. Thus, in cancer diagnosis, it is necessary to conduct pathological diagnosis of a biopsy specimen for definite diagnosis and to evaluate abnormal cells at the cellular level. In pathological diagnosis of a biopsy specimen, however, especially earlier cancers provide a higher possibility of overlooking cancer cells or cells in a precancerous condition, that is, a higher possibility of diagnosing as negative by mistake (false negative). Particularly when taking out only a small portion of tissue for biopsy in internal medical examination, it is a challenge to find a means of decreasing a risk that such false negative diagnosis is given, and that the disease state fully progresses before first awakening thereof. The same problem produces a large challenge also in evaluation of presence or absence of postoperative recurrence, and in a large number of clinical departments facing ever-increasing cancer patients, there is a need for a properly diagnosing method that is not significantly depending on the level of proficiency and experiences of an attending doctor.

Biopsy includes a case of conducting for confirmatory purpose during surgical operation, a case of conducting from the inside of a digestive tract, trachea, urinary bladder, vagina and other various lumens in internal medical examination using an endoscope, a bronchoscope, a mesoscope, a rhinoscope, or an otoscope, or a case in which a small hole is made on the body near areas of interest, and an abdominoscope or a thoracoscope is inserted into an opening and biopsy is done from the outside, however, it is extremely important to select a precise biopsy site in all these cases since these are invasive operations. In an endoscopic examination, when a region showing reddening, erosion, white protuberance or abnormality in fine vascular structure is recognized, a biopsy site is selected by detailed examination of such as an atypical vessel pattern using a magnifying endoscope. In the case of micro cancers, however, it often becomes impossible to find the lesioned part if the biopsy site is mistaken, due to bleeding in biopsy. In contrast, if tissue which is difficult to be distinguished from normalcy is extensively excised, the degree of invasion increases. Also in cancer operations, unnecessarily large excision of a normal part to avoid recurrence and the follow-up surgery acts as a minus factor in view of an improvement in QOL (Quality of Life). Therefore, in surgical operations, a process, in which from a part which seems to securely contain a cancer to a part remote by a certain distance are once excised and rapid pathological diagnosis of the amputation stump is conducted, is repeated during the operation, to check if the excised range is suitable or not. However, it takes several tens of minutes to 1 hour per an amputation stump from submitting the stump to rapid pathological diagnosis until obtaining the result thereof, thus, it is one of factors inhibiting reduction of the operation time. An extremely heavy burden rests on a patient during diagnosis not only at the time of surgery but also in biopsy performed in an examination with an endoscope. Therefore, there is an ardent need in the medical front for an imaging method to display which part should be examined in detail and should be subjected to biopsy, for terminating the examination as quickly as possible. In cancers for which the time of finding thereof in an examination is often already too late such as biliary tract cancer and Barrett's esophageal cancer, an improvement in survival rate by early detection is expected and an imaging method capable of finding a small cancer correctly and easily is required. Further, once a cancer is found, the degree of spreading of the cancer superficially present on the surface layer of the lumen (referred to as lateral spreading or horizontal extension) should be properly determined and utilized in therapy thereof, however, it is difficult for even proficient experts to conduct this at the cellular level with an endoscope (non-patent document 1).

Currently, various endoscopes such as those of high resolution, those of Narrow Band Imaging (NBI) utilizing a change of light scattering and absorption, those imaging cell autofluorescence (AFI), and those using a new principle such as Optical Coherence Tomography (OCT) are being developed, for capturing and observing more clearly the feature of a cancer. Of them, usefulness of NBI is recognized and it is becoming widespread rapidly. However, NBI is a method for indirectly evaluating the degree of cancer progression mainly by visualizing a blood vessel pattern, and thus is unsuitable for direct observation of individual cells, like the other methods exemplified previously. By contrast, in the case of a confocal endoscope (Confocal Laser Endomicroscopy, non-patent document 2), a fluorescent molecule such as fluorescein is intravenously injected and the morphological feature of each cell can be observed by acquiring a tomographic image using fluorescence emitted from the tissue, thus, if sensitivity thereof is enhanced, determination of a difference in dysplasia and degree of malignancy at the cellular level is expected. Application thereof at the time of surgery is also possible. However, since cellular uptake of fluorescein is not cancer cell-specific, it needs an immense amount of time to precisely examine fluorescence of cancer cells exhibiting abnormality during endoscopy or other examinations, under conditions in which almost all cells are emitting fluorescence in the tissue, which is huge compared to the confocal endomicroscopic field. Hence, there is a need for a suitable contrast agent which rapidly detects cancer cells and cells in a precancerous condition and enables discrimination from cell abnormalities ascribable to causes other than cancer such as inflammation (non-patent document 3 and non-patent document 4).

One of the most frequently used contrast agents enabling cancer diagnosis imaging at present is a 2-deoxy-D-glucose derivative radioactively labeled with ¹⁸F: [¹⁸F]2-fluoro-2-deoxy-D-glucose (FDG), and clinical image diagnosis combining this with positron emission tomography (PET) is conducted widely (non-patent document 5). The FDG method visualizes a cancer by utilizing that cancer cells take up a larger amount of D-glucose into the cell than normal cells, however, has a defect that uptake of D-glucose into single living cells cannot be continuously monitored in real time for methodological reasons of measurement (non-patent document 6). This is a disadvantage in proper diagnosis of cancers scattered in normal cells which show non-uniform morphologies, functions and degrees of differentiation, and is one of the factors making early detection difficult of for example extremely small cancer scattered and cancers generated superficially in the mucosa of a digestive organ. Moreover, there is a fundamental problem that background signals being generated by FDG uptake into normal cells surrounding cancer tissue, or in some cases within cancer tissue, lower the SN (signal-to-noise) ratio, since D-glucose is taken up into normal cells as well. Furthermore, since FDG readily concentrates on inflammatory cells, discrimination from a cancer is difficult.

On the other hand, the present inventors have indicated that 2-amino-2-deoxy-D-glucose labeled with a 7-nitrobenz-2-oxa-1,3-diazole group (NBD) as a fluorescent group, which is abbreviated as 2-NBDG, is taken up into mammalian cells through a glucose transporter thereof with kinetics similar to a radiolabeled D-glucose derivative (non-patent document 6). Based on these backgrounds, there are suggestions that a cancer is imaged at the cellular level using a fluorescently labeled D-glucose derivative such as 2-NBDG instead of FDG (non-patent document 7 and patent document 1). Further, as imaging examples of 2-NBDG application to biopsy specimens or surgical specimens excised from cancer tissues, those applied to oral cavity cancer (non-patent document 8), breast cancer (non-patent document 9) and Barrett's esophageal cancer have been reported (non-patent document 1).

When 2-NBDG is used for cancer diagnosis imaging, however, background signals attributable to the uptake into normal cells lower the SN ratio, making early detection of micro cancers difficult just like FDG, since 2-NBDG is a D-glucose derivative. Actually, D-glucose shows a nature of being taken up intensively into an adipocyte and muscle when blood glucose level is elevated (non-patent document 10), and FDG shows the same nature as well. Therefore, in the PET examination using FDG, fasting for a long period of time before the examination is essential for increasing the SN ratio, and during the period prior to FDG uptake (about 30 minutes to about 1 hour) and during the measurement period (about 20 to 30 minutes), a patient should keep his body still without moving muscle (conversation is restricted as well). Likewise, fasting is necessary in cancer imaging with 2-NBDG as well (non-patent document 11). However, even if fasting is carried out, uptake of a fluorescently labeled D-glucose derivative into normal cells cannot be suppressed completely, thus, a detection method with higher accuracy is required. Recently, it has been reported that mild dysplastic cells showing relatively weak uptake of 2-NBDG and severe dysplastic cells taking up 2-NBDG intensively are included in human tissue biopsy specimens (non-patent document 1), however, since 2-NBDG is taken up into any of these, discrimination of them has to be done continuously. Moreover, since inflammatory tissues take up 2-NBDG intensively as well, to discriminate these from cancer is a challenge as in the FDG-PET method (non-patent document 1). Therefore, as long as a fluorescently labeled D-glucose derivative is used, one can say that accurate determination of tumor cells is limited. Furthermore, it is difficult to diagnose cancer in diabetic patients by the FDG method, and the age at higher risk of developing cancer and the onset age of diabetes well overlap, and thus, imaging with 2-NBDG may face similar restrictions.

In addition, there is a recent suggestion on a method of synthesizing a derivative prepared by linking a fluorescent molecular group to the 1-position of D-form or L-form glucose via a linker, and this derivative is applied to normal cells and specific tumor cells and fluorescence detection thereof is performed (patent document 3). However, examples of the patent document 3 show that a sufficient increase in fluorescence intensity due to an L-form glucose derivative is detected in any of normal cells and tumor cells.
2-NBDG and 2-NBDLG are described in WO 2010/016587 (patent document 2) regarding the prior study results of the present inventors . Of them, 2-NBDLG is believed not to pass though GLUT as a glucose transporter (non-patent document 15, patent document 2). In the method of evaluating specific uptake of D-glucose into a cell suggested in the patent document 2, 2-NBDG is used as the fluorescently labeled D-glucose derivative.

### PRIOR ART DOCUMENTS

### Patent Document

Patent Document 1: US Patent No. 6989140 Specification
Patent Document 2: WO2010/016587 Publication
Patent Document 3: US Patent Application Publication No. 20090317829

### Non-Patent Document

Non-patent Document 1: Thekkek et al., Technol. Cancer Res. Treat. 10: 431-41, 2011
Non-patent Document 2: Hsiung, PL. et al., Nat. Med. 14: 454-8, 2008
Non-patent Document 3: Thekkek & Richards-Kortum, Nat. Rev. Cancer 8: 725-31, 2008
Non-patent Document 4: Goetz & Wang., Gastroenterol. 138: 828-33, 2010
Non-patent Document 5: Jadvar et al., J. Necl. Med. 50: 1820-27, 2009
Non-patent Document 6: Yamada et al., J. Biol. Chem. 275: 22278-83, 2000
Non-patent Document 7: O'Neil et al., Mol. Imaging Biol. 7: 388-92, 2005
Non-patent Document 8: Nitin et al., Int. J. Cancer 124: 2634-42, 2009
Non-patent Document 9: Langsner et al., Biomed. Optics Express 2: 1514-23, 2011
Non-patent Document 10: Foley et al., Biochemistry 50: 3048-61, 2011
Non-patent Document 11: Sheth et al., J. Biomed. Optics 14: 064014, 2009
Non-patent Document 12: Thompson RJ. et al., Science 312, 924-927,2006.
Non-patent Document 13: Cheng et al., Bioconjugate Chem. 17: 662-69, 2006
Non-patent Document 14: Etxeberria et al., J. Experimental Botany 56:1905-12, 2005
Non-patent Document 15: Yamamoto et al., Tetrahedron Lett. 49: 6876-78, 2008
Non-patent Document 16: Yamada et al., Nat. Protoc. 2: 753-762, 2007

### SUMMARY OF THE INVENTION

### PROBLEM TO BE SOLVED BY THE INVENTION

That is, the present invention has an object of providing a method of accurately detecting cancer cells by imaging, and an imaging agent used in this method.

### MEANS FOR SOLVING THE PROBLEM

After intensive studies in view of the above-described points, the present inventors have found that developed tumor cells (for example, cells constituting a tumor cluster at a stage so developed as to include many cells exhibiting abnormal cell nuclei in the cell cluster) take up a fluorescently labeled L-glucose derivative actively, and completed the present invention.

The present invention provides a method of detecting that the target cells are cancer cells *in vitro,* using an L-glucose derivative having a specific fluorescent chromophore group (7-nitrobenz-2-oxa-1,3-diazole group or 7-(N,N-dimethylaminosulfonyl)benz-2-oxa-1,3-diazole group) in the molecule and of performing determination thereof. The present invention also provides such L-glucose derivative for use in an *in vivo* diagnosing method for detecting cancer cells. More specifically, the present invention is as described below.
(1) A method of detecting cancer cells *in vitro,* comprising the following steps:
   (a) a step of bringing a composition containing an L-glucose or an L-glucose in which a hydroxyl group is substituted by an amino group or a fluorine atom, said L-glucose being linked to a 7-nitrobenz-2-oxa-1,3-diazole group or a 7-(N,N-dimethylaminosulfonyl)benz-2-oxa-1,3-diazole group via an amine or via a spacer represented by the formula -NH- (CH₂)ₙ-NH-, in which formula n is 2 to 20, or a salt form thereof into contact with a target cell *in vitro,*
   (b) a step of detecting the L-glucose linked to the 7-nitrobenz-2-oxa-1,3-diazole group or 7-(N,N-dimethylaminosulfonyl)benz-2-oxa-1,3-diazole group present in the target cell by detecting fluorescence, and
   (c) a step of detecting a cancer cell based on an increase in the fluorescence intensity in the target cell.
(2) The detection method according to (1) wherein the above-described L-glucose linked to a 7-nitrobenz-2-oxa-1,3-diazole group or a 7-(N,N-dimethylaminosulfonyl)benz-2-oxa-1,3-diazole group is obtained by linking a 7-nitrobenz-2-oxa-1,3-diazole group or a 7-(N,N-dimethylaminosulfonyl)benz-2-oxa-1,3-diazole group to the 2-position, 4-position or 6-position of the L-glucose or L-glucose in which a hydroxyl group is substituted by an amino group or a fluorine atom.
(3) The detection method according to (2) wherein the above-described L-glucose linked to a 7-nitrobenz-2-oxa-1,3-diazole group or a 7-(N,N-dimethylaminosulfonyl)benz-2-oxa-1,3-diazole group is 2-[N-(7-nitrobenz-2-oxa-1,3-diazole -4-yl)amino]-2-deoxy-L-glucose or 2-deoxy-2-[N-7-(N',N'-dimethylaminosulfonyl)benz-2-oxa-1,3-diazole-4-yl)amino]-L-glucose.
(4) The detection method according to any one of (1) to (3) wherein detection in the above-described step (b) is conducted by imaging the target cell.
(5) The detection method according to any one of (1) to (4) wherein the composition in the above-described step (a) further contains another L-glucose or another L-glucose in which a hydroxyl group is substituted by an amino group or a fluorine atom, said another L-glucose being linked to sulforhodamine, wherein the L-glucose of the composition of step (a) and the said another L-glucose are differently taken up into cells and detected at different wavelengths, thereby excluding noises by the uptake attributable to cell membrane damages due to inflammation or physical injury, and by non-specific uptake of macrophage.
(6) The detection method according to (5) wherein the above-described composition contains 2-[N-(7-nitrobenz-2-oxa-1,3-diazole-4-yl)amino]-2-deoxy-L-glucose and 2-amino-2-deoxy-L-glucose linked to sulforhodamine 101 or sulforhodamine B at the 2-position by way of a sulfonamide bond.
(7) The detection method according to (5) or (6) wherein detection in the above-described step (b) is conducted by using a temporal change in the fluorescent color tone of the imaged cell as an index.
(8) The detection method according to any one of (1) to (7) wherein the above-described all steps are carried out within 30 minutes.
(9) A compound for use in an *in vivo* diagnosing method for detecting cancer cells, wherein said compound is an L-glucose or an L-glucose in which a hydroxyl group is substituted by an amino group or a fluorine atom, said L-glucose being linked to a 7-nitrobenz-2-oxa-1,3-diazole group or a 7-(N,N-dimethylaminosulfonyl)benz-2-oxa-1,3-diazole group via an amine or via a spacer represented by the formula -NH- (CH₂)ₙ-NH-, in which formula n is 2 to 20, or a salt form thereof, wherein the diagnosing method comprises the steps of:
   (1) administering said compound to a living body locally or systemically,
   (2) performing imaging, and
   (3) evaluating the uptake of said compound into a cell by detecting an increase in the fluorescence intensity, and thus detecting cancer cells..
(10) The compound for use according to (9) wherein the above-described L-glucose linked to the 7-nitrobenz-2-oxa-1, 3-diazole group or 7-(N,N-dimethylaminosulfonyl)benz-2-oxa-1,3-diazole group is obtained by linking a 7-nitrobenz-2-oxa-1,3-diazole group or a 7-(N,N-dimethylaminosulfonyl)benz-2-oxa-1,3-diazole group to the 2-position, 4-position or 6-position of L-glucose or L-glucose in which a hydroxyl group is substituted by an amino group or a fluorine atom.
(11) The compound for use according to (10) wherein the above-described L-glucose linked to the 7-nitrobenz-2-oxa-1, 3-diazole group or the 7-(N,N-dimethylaminosulfonyl)benz-2-oxa-1,3-diazole group is 2-[N-(7-nitrobenz-2-oxa-1,3-diazole-4-yl)amino]-2-deoxy-L-glucoseor 2-deoxy-2-[N-7-(N',N'-dimethylaminosulfonyl)benz-2-oxa-1,3-diazole-4-yl)amino]-L-glucose.
(12) The compound for use according to any one of (9) to (11) wherein the imaging agent further contains another L-glucose or another L-glucose in which a hydroxyl group is substituted by an amino group or a fluorine atom, said another L-glucose being linked to sulforhodamine.
(13) The compound for use according to (12) wherein the L-glucose linked to sulforhodamine is 2-amino-2-deoxy-L-glucose linked to sulforhodamine 101 or sulforhodamine B at the 2-position by way of sulfonamide bond.
(14) The compound for use according to (9) wherein the above-described L-glucose linked to a 7-nitrobenz-2-oxa-1,3-diazole group or a 7-(N,N-dimethylaminosulfonyl)benz-2-oxa-1,3-diazole group is 6-[¹⁸F]-2-[N-(7-nitrobenz-2-oxa-1,3-diazole-4-yl)amino]-2-deoxy-L-glucose or 4-[¹⁸F]-2-[N-(7-nitrobenz-2-oxa-1,3-diazole-4-yl)amino]-2-deoxy-L-glucose.
(15) The compound for use according to (14) for use in an *in vivo* diagnosing method for confirming the site of cancer cells with a whole body imaging using PET.

### EFFECT OF THE INVENTION

The present invention provides an *in vitro* method and a compound for use in an *in vivo* diagnosing method for detecting cancer cells, which are capable of discriminating cancer cells at a high contrast.

### BRIEF EXPLANATION OF DRAWINGS

Fig. 1 is a photograph showing the results of Example 2 in which tumor cells (C6 glioma) were transplanted subcutaneously at the root of right hind paw of a mouse and 2-NBDLG was administered to the mouse and imaging was performed every one minute. An arrow denotes a time point at which 2-NBDLG was administered.
Fig. 2 is a photograph showing the results of imaging after administration of 2-NBDG in Example 2. An arrow denotes a time point at which 2-NBDG was administered.
Fig. 3 is a graph showing the temporal change in the accumulated value at the tumor cell-transplanted part (circle at root part of right posterior leg, rhombus in graph, Tumor+Glc) and at a dorsal adipocyte-rich part near neck (circle at neck part, square in graph, BG+Glc) after administration of 2-NBDLG and 2-NBDG in Example 2.
Fig. 4 is an image acquired by a real time confocal laser scanning microscope after administration of 2-NBDLG to a tumor cell cluster composed of mouse insulinoma cells (MIN6) in Example 3.
Fig. 5 is an image acquired by a real time confocal laser scanning microscope before administration of a mixed solution composed of 100 µmol of 2-NBDLG and 20 µmol of 2-TRLG to a tumor cell cluster composed of mouse insulinoma cells (MIN6) in Example 4.
Fig. 6 is an image acquired 2 minutes after cessation of administration in Example 4.
Fig. 7 is an image acquired 4 minutes after cessation of administration in Example 4.
Fig. 8 is an image obtained by overlaying the green channel image, the red channel image and the DIC image 2 minutes after cessation of administration in Example 4.
Fig. 9 is an image obtained by overlaying the green channel image, the red channel image and the DIC image 4 minutes after cessation of administration in Example 4.
Fig. 10 shows the results revealing an effect of an inhibitor on the uptake of 2-NBDG and 2-NBDLG into mouse insulinoma (MIN6) cells.
Fig. 11 is an image acquired by a real time confocal laser scanning microscope before administration of a mixed solution composed of 100 µmol of 2-NBDLM and 20 µmol of 2-TRLG to acutely dissociated neurons in Example 6.
Fig. 12 is an image acquired at administration in Example 6.
Fig. 13 is an image acquired 2 minutes after cessation of administration in Example 6.
Fig. 14 is an image acquired 10 minutes after cessation of administration in Example 6.
Fig. 15 is an image acquired 22 minutes after cessation of administration in Example 6.

### DESCRIPTION OF EMBODIMENTS

The present invention relates to a method of detecting cancer cells *in vitro* using a fluorescently labeled L-glucose derivative, and said compound for use in an in vivo diagnosing method for detecting cancer cells. According to the present invention, it is possible to determine whether the targeted cells are cancer cells or not by bringing a composition containing an effective amount of a fluorescently labeled L-glucose derivative (an L-glucose or an L-glucose in which a hydroxyl group is substituted by an amino group or a fluorine atom, said L-glucose being linked to a 7-nitrobenz-2-oxa-1,3-diazole group or a 7-(N,N-dimethylaminosulfonyl)benz-2-oxa-1, 3-diazole group via an amine or via a spacer represented by the formula -NH-(CH2)n-NH-, in which formula n is 2 to 20, or a salt form thereof, wherein said 7-nitrobenz-2-oxa-1,3-diazole group or 7-(N,N-dimethylaminosulfonyl)benz-2-oxa-1,3-diazole group is the fluorescent molecular group in the molecule), as a reagent, into contact with the target cells. Further, according to the present invention, it is possible to detect cancer cells in tissue by bringing the composition containing the fluorescently labeled L-glucose derivative into contact with the tissue containing the target cells and imaging the cells. Furthermore, according to the present invention, it is possible to detect cancer cells or tissue containing these cells by administering the composition containing the fluorescently labeled L-glucose derivative to a living body and performing imaging, and this method is useful as a method of detecting a cancer. The fluorescently labeled L-glucose derivative is not taken up or if any, taken up in a trace amount in a short period of time such as, for example, within 20 minutes after initiation of administration, into normal cells excepting special cells presenting phagocytosis such as macrophage and microglia, thus, according to the present invention, it is possible to conduct imaging in a short period of time (for example, within 15 minutes after initiation of administration), and additionally, it is possible to obtain high contrast between cancer cells and normal cells or cells constituting a tumor cell cluster not so developed as to cause abnormalities in nuclei.

In administering fluorescently labeled D-glucose, fasting is usually carried out before administration for the purpose of lowering the uptake into adipocytes and muscle, however, when fasting is conducted, hemichannel (gap junction/hemichannel) openings occur and uptake of D-glucose via hemichannels may lower contrast, depending on the organ (non-patent document 12). In fasting, normal cells in muscle, liver, pancreas, kidney start autophagy. Autophagy is a phenomenon that normal cells digest themselves, and if autophagy occurs in various cells, a possibility that a fluorescently labeled D-glucose derivative is accordingly taken up non-specifically into normal cells other than cancer cells cannot be denied. As described above, fasting exerts a negative effect on identification of cancer cells by imaging in some cases. Also when applied to *in vitro* evaluation of live cells, if cells are maintained for a certain period without adding saccharides utilizable as an energy source such as glucose, analogous non-specific uptake possibly occurs. Further, it is suggested that if D-glucose or a derivative prepared by linking a fluorescent group to D-glucose is kept in contact with a normal cell for a long period of time (specifically, 30 minutes or longer), membrane proteins such as glucose transporters or glucose receptors present on the plasma membrane of the normal cell transfer into the cytosol with the derivative binding to them (internalization) (non-patent document 13), or non-specific endocytosis possibly occurs (non-patent document 14). In contrast, in the present invention, imaging can be carried out in a short period of time (for example, within 15 minutes after starting administration) without fasting in a living body, and according to the present invention, higher contrast can be obtained between cancer cells and normal cells as compared with imaging using a labeled D-glucose derivative. Since fasting is not required to be effected in detection and determination, a patient is not forced to bear the burden and an examination can be carried out immediately.

The term "cancer cells" as used in the present specification denotes cells showing abnormality in cell morphology (structural atypia) or cells constituting a tumor cluster together with such cells, cells showing abnormality in nucleus morphology (nuclear atypia) and abnormality in nucleus division, or cells having a high possibility of progressing into such cells, and in general, denotes cells classified into malignant tumors and diagnosed as a cancer, or cells constituting such cell populations, or cells having a high possibility of progressing into such cell populations.

In a living body, though not cancer cells, there are cells having a possibility of taking up the fluorescently labeled L-glucose derivative of the present invention non-specifically into the cytosol by phagocytosis as exemplified by macrophage and microglia. These cells, though they are neither cancer cells nor cells of broken plasma membrane, may take up a fluorescently labeled L-glucose derivative. The type of these cells can be identified either by their morphology or by their cellular uptake of 2-TRLG when administered simultaneously, or by using a specific marker like CD14 or CD11b, after being determined as false-positive by the method of the present specification.

Though the kind of cancer cells which can be determined is not particularly restricted, examples thereof include cancer cells in cancers in the ophthalmologic field such as of eyelid, lacrimal gland, cancers of ear parts such as outer ear, middle ear, cancers of nose parts such as nasal cavity, paranasal cavity, lung cancer, digestive organ cancers such as oral cavity cancer, larynx cancer, pharynx cancer, esophageal cancer, stomach cancer, small intestinal cancer, large intestine cancer, cancers in the gynecological field such as breast cancer, uterus cancer, ovary cancer, fallopian tube cancer, cancers of genital organs, kidney cancer, urinary bladder cancer, prostate cancer, anus cancer, skin cancer, bone cancer, muscle cancer (sarcoma), blood cancers such as leukemia, malignant lymphoma, cancers of peripheral and central nerves, and glia cancer.

The "fluorescently labeled L-glucose derivative" used in the present invention is a L-glucose derivative having a 7-nitrobenz-2-oxa-1,3-diazole group (NBD)or a 7-(N,N-dimethylaminosulfonyl)benz-2-oxa-1,3-diazole group (DBD) in the molecule. Though the position of linking of NBD in the L-glucose derivative is not particularly restricted, the position includes, specifically, the 1-position, 2-position, 3-position, 4-position or 6-position, preferably the 2-position, 4-position or 6-position, further preferably the 2-position or 6-position, most preferable the 2-position of L-glucose. NBD or DBD may be directly linked to L-glucose via an amine, or may be linked via a spacer represented by the formula -NH- (CH₂)ₙ-NH-(here, n is 2 to 20, preferably 2 to 10). The L-glucose derivative having a 7-nitrobenz-2-oxa-1,3-diazole group or a 7-(N,N-dimethylaminosulfonyl)benz-2-oxa-1,3-diazole group emitting green fluorescence in the molecule shows the maximum fluorescence wavelength around 530 to 580 nm.

The fluorescently labeled L-glucose derivative contains L-glucose or L-glucose in which a hydroxyl group is substituted by an amino group or a fluorine atom. It may also be a salt form such as a hydrochloride.

The fluorescently labeled L-glucose derivative which can be used in the present invention includes, specifically, L-glucose derivatives having a 7-nitrobenz-2-oxa-1,3-diazole group emitting green fluorescence in the molecule (maximum fluorescence wavelength thereof is around 530 nm to 580 nm), for example, 2-[N-(7-nitrobenz-2-oxa-1,3-diazole-4-yl)amino]-2-deoxy-L-glucose(2-NBDLG), 4-deoxy-4-[N-(7-nitrobenz-2-oxa-1,3-diazole-4-yl)amino]-L-glucose(4-NBDLG), 6-deoxy-[N-(7-nitrobenz-2-oxa-1,3-diazole-4-yl)amino]-L-glucose(6-NBDLG). Further mentioned are L-glucose derivatives having a (N,N-dimethylaminosulfonyl)benz-2-oxa-1,3-diazole group emitting yellow to yellow-green fluorescence as a derivative of NBD in the molecule, for example, 2-deoxy-2-[N-7-(N',N'-dimethylaminosulfonyl)benz-2-oxa-1,3-diazole-4-yl)amino]-L-glucose(2-DBDLG).

Some of these compounds are described in WO 2010/016587 published bulletin (patent document 2) regarding the prior study results of the present inventors. Of them, 2-NBDLG has an enantiomeric relation with 2-NBDG (2- [N- (7-nitrobenz-2-oxa-1,3-diazole-4-yl)amino]-2-deoxy-D-glucose) as shown below, and is believed not to pass through GLUT as a glucose transporter (non-patent document 15, patent document 2). 2-NBDG is a compound suggested by the present inventors for study of a process of dynamic uptake of D-glucose into a live mammal cell, and currently regarded as indispensable in this study field (non-patent document 16). Also in the method of evaluating specific uptake of D-glucose into a cell suggested in the patent document 2, 2-NBDG is used as the fluorescently labeled D-glucose derivative.

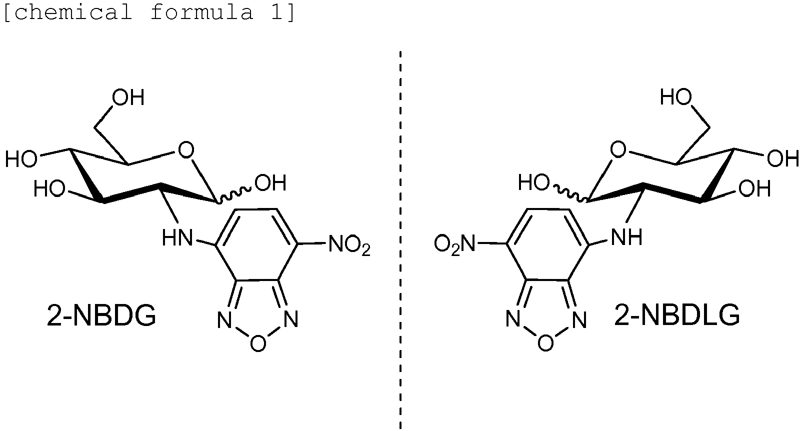

The fluorescently labeled L-glucose derivative used in the present invention is a L-glucose derivative having NBD or DBD in the molecule, preferably 2-NBDLG or 2-DBDLG, further preferably 2-NBDLG. The L-glucose in which a hydroxyl group is substituted by an amino group or a fluorine atom includes, for example, L-glucose in which a hydroxyl group is substituted by an amino group, L-glucose in which a hydroxyl group is substituted by an amino group and a hydroxyl group is substituted by a fluorine atom, and L-glucose in which a hydroxyl group is substituted by a fluorine atom, further, for example, molecules obtained by introducing fluorine at the 2-position, 3-position, 4-position, 6-position of L-glucose, for example, 2-F-6-NBDLG, 3-F-2-NBDLG, 4-F-2-NBDLG, 6-F-2-NBDLG.

The target cell is not particularly restricted and may include any cells such as cells isolated from a living body, cells present in tissue isolated from a living body, cells present in tissue of a living body, primary cultured cells after isolation from a living body, and established cells.

The fluorescently labeled L-glucose derivative is taken up into a cancer cell and detected in the cell. Detection of the L-glucose derivative in a cancer cell can be carried out by a usually used method for detecting fluorescence. For example, it can be conducted as described below.

The detection of a fluorescently labeled L-glucose derivative present in a cancer cell can be evaluated as follows: the fluorescence of the target cell is measured beforehand, then a fluorescently labeled L-glucose derivative is kept in contact with the target cell for a certain period of time, subsequently this is washed away, the fluorescence of the target cell is again measured, and the evaluation is made according to an increase in the fluorescence intensity with respect to the fluorescence intensity of the target cell before contact. Imaging of cells having the fluorescently labeled L-glucose derivative in the cell is made in such a way that the fluorescence intensity is recognized as an image, and thus, cancer cells can be detected. The evaluation may also be performed by the sum of fluorescence intensities or distribution of fluorescence intensities manifested from a large amount of cells using a fluorescence plate reader or a flow cytometry.

Detection of fluorescence can be carried out at any time after cessation of washout. When an L-glucose derivative is brought into direct contact with tissue or cells isolated from a living body, detection can be performed, for example, from just after cessation of washout to 20 minute after, preferably from just after cessation of washout to 10 minute after. When the target cell is a cell present in tissue and it takes a long time in washout of an L-glucose derivative in the extracellular tissue around the cell of interest, detection can be performed after completion thereof. Detection can be performed with a good contrast after waiting for a certain period from the contact, however, in case of using a detection apparatus capable of acquiring the tomographic image of a cell such as a confocal microscope or a multiphoton microscope, it is also possible to detect fluorescence intensity or a change of fluorescence intensity in the target cell during the contact period and to evaluate these in comparison with those before initiation of the contact and/or imaged. As described above, according to the present invention, a process for detecting cancer cells is completed within 30 minutes, preferably within 15 minutes directly after starting contact of the composition containing the fluorescently labeled L-glucose derivative to the cell. Further, according to the present invention, cancer cells can be detected within several minutes for isolated cells, and within 10 minutes even for cells present in tissue after starting contact of the fluorescently labeled L-glucose derivative to the cell, in many cases. When the target cell is a cell in a living body, the imaging agent is brought into direct contact with the living body locally, then, washed away and fluorescence is detected. Alternatively, the imaging agent is administered into a vessel such as a vein, and detection is performed after the agent reaches a region including the target cell. When administered into a vessel, a special procedure for washout is not necessary. Furthermore, when the fluorescently labeled L-glucose derivative is administered into a vessel such as a vein, systemic imaging can also be carried out. These cases are described specifically below.

When the target to which an effective amount of a fluorescently labeled L-glucose derivative is administered is a cell in a living body, it may be recommended for example that the labeled L-glucose derivative is dissolved in physiological saline for injection to prepare a liquid reagent, in use, and the reagent is intravenously injected. When a region for the test is on the skin surface or on the inner side of a lumen such as oral cavity, digestive tract, urinary bladder, or vagina, a liquid reagent may be directly coated, dropped or sprayed on the region for the examination, or a gelled reagent is allowed to contact for a certain period such as, for example, several minutes. When applied to urinary bladder, it may also be permissible that urine is removed beforehand, and then a liquid reagent is injected transurethrally to attain contact. When mucus blocks effective contact in digestive tracts, it is also possible that mucus is treated and removed by pronase as usually conducted in an endoscopic examination, and contact is attained by a method known per se like in the case of spraying indigo carmine or acetic acid. In this case, it is possible that an excess liquid reagent remaining on the region for the examination is washed away before conducting imaging, and uptake into a cell is evaluated in terms of an increase in fluorescence intensity with respect to the fluorescence image on the region for the examination before contact of the reagent. However, even when an excess liquid reagent remains on the region for the examination, if a suitable fluorescent microscopic observation apparatus having a confocal effect (for example, confocal endoscope) as described above is used and observation is performed by adjusting the focal depth to a cell of interest, then it is possible to know during the contact period whether or not the labeled L-glucose derivative is taken up into the cell which maintains the membrane integrity. Imaging may advantageously be conducted by an imaging method known per se corresponding to a labeled fluorescence of the fluorescently labeled L-glucose derivative. According to the present invention, tumor cells and normal cells can be discriminated with higher contrast several minutes after administration of the labeled L-glucose derivative into a mouse vain, as compared with a case of use of a labeled D-glucose derivative. Because of the above-described constitution, timing of evaluation by imaging is within 40 minutes, preferably within 20 minutes, further preferably within 10 minutes directly after staring administration of the labeled L-glucose derivative to a living body. Evaluation by imaging may be conducted only once at specific time point, or conducted at several time points and evaluation may be carried out based on the temporal change in the contrast between cells of interest.

In case when the target cell is in tissue or a cell sampled from a living body, the tissue and a cell obtained by biopsy, at the starting of a surgical operation, or during an operation are used as specimens, and the specimens are immersed in a liquid reagent prepared by dissolving the fluorescently labeled L-glucose derivative in physiological saline for injection, the liquid reagent is dropped on the specimens, or the specimens are perfused with the liquid reagent, then, an excess liquid reagent remaining on the outside of the specimen is washed away, then, imaging is conducted, thereby detecting the labeled L-glucose derivative in the cell. This method is different from the above-described method because the specimen is taken out from a living body, and it is necessary, for example, to provide a method known per se for recovering tissue in which cut surface has been injured when taken out, by placing the tissue in a Ringer's solution for a certain period, however, the kind of the fluorescently labeled L-glucose derivative to be used and the imaging principle may be the same as in the above-mentioned method excepting the above-described recovering procedure. Also these imaging steps are completed within 30 minutes, preferably within 15 minutes directly after starting contact of the labeled L-glucose derivative to tissue or a cell, and cancer cells can be detected within several minutes for isolated cells and within 10 minutes even for cells in tissue after starting contact of the labeled L-glucose derivative to a cell, in many cases.

When the fluorescently labeled L-glucose derivative is used for *in vivo* diagnosis, application to intraoperative assessment and endoscopic diagnosis is possible, and as the method of administration, systemic administration methods such as an intravenous administration as described previously, and additionally, direct administration of minimally required amount to an affected part, are envisaged. Specifically, a method of directly coating, dropping or spraying on an affected part or the surgical field, or a method of local administration by spray in an examination using an endoscope, and a method of contacting a gelled reagent are envisaged.

When radioactive labeling is used in addition to fluorescent labeling, detection of cancer cells can be conducted by detecting radiation in addition to fluorescence, for example by PET detecting gamma-ray.

When the fluorescently labeled L-glucose derivative is used for *in vivo* diagnosis and if cancer or precancerous lesion is suspected by the imaging, tissue biopsy is then carried out, the sample is fixed with formalin, then, a pathological examination is carried out to obtain a definite diagnosis. Reliability in selecting a biopsy site can be improved by prior imaging. Further, the fluorescently labeled L-glucose derivative for use *in in vivo* cancer diagnosis can also be used for intraoperative assessment in combination with an endoscope and other fluorescence observation apparatuses, and if the labeled L-glucose derivative is used as an aid for properly determining the range of surgical excision, it is expected that the burden on a patient and a risk are reduced due to a shortening of the operation time, and that QOL and prognosis are improved.

Worthy of mention in the present invention is that it informs us of the extent of tumor cell progression based on the uptake degree of the labeled L-glucose derivative into the cell. Specifically, the present inventors confirmed that the tumor cells actively taking up the labeled L-glucose derivative are cells making up a developed tumor cluster wherein a significant portion of cells bear abnormal nucleus, that is, cancer cells constituting malignant tumors. Therefore, if the labeled L-glucose derivative is detected in cells, it can be determined that the cells are cancer cells constituting a developed tumor cell cluster.

The L-glucose derivative, by virtue of the fact that it is L-form, plays a role in showing whether or not a membrane protein like GLUT, which has stereoselectivity for glucose, preserves a mechanism to properly transport its ligand in a stereoselective manner. As exemplified by tumor cells, in the case of cells which express both stereoselective membrane transport mechanisms for glucose (mechanisms allowing selective transport of only one form of glucose in two mirror image D- and L-isomers) and non-stereoselective membrane transport mechanisms for glucose (mechanisms mediated by a membrane protein allowing transport of both D-form and L-form isomers, excluding permeation due to breakdown of a lipid bilayer structure of plasma membrane as typified by permeation of a membrane-impermeable marker such as propidium iodide) in parallel, it is possible to discriminate them from normal cells, or to discriminate the degree of malignancy or the difference in cell conditions due to micro-environmental factors even among tumor cells of the same kind, based on the degree of contribution of transport via the latter pathway to the whole transport amount.

The imaging agent can further contain an L-glucose derivative having a fluorescent chromophore group of different wavelength in addition to the fluorescently labeled L-glucose derivative used in the present invention. By this, whether cells are cancer cells or not can be more correctly determined by bringing the fluorescently labeled L-glucose derivative used in the present invention and the other fluorescently labeled L-glucose derivative detected at different wavelengths into contact with the cells and by measuring the change in the color tone of the imaged cells depending upon the elapsed time as an index. Specifically, by using both the fluorescently labeled L-glucose derivative used in the present invention and the discriminatable fluorescently labeled L-glucose derivative simultaneously, it is possible to evaluate the condition of individual cancer cells with continuous fluorescent color depicted by the proportion of wavelength components of these derivatives, based on a difference in the uptake of these L-glucose derivatives into cells and a difference in the elimination time course of the L-glucose derivatives from the cell, and it is possible to exclude noises by the uptake attributable to cell membrane damages due to inflammation or physical injury, and by non-specific uptake of macrophage. That is, whether the target cells are cancer cells or not can be determined with a high accuracy based on the change in the fluorescent color tone as described above.

For example, the imaging agent can further contain L-glucose derivatives containing red fluorescence-emitting sulforhodamine (sulforhodamine B, sulforhodamine G, sulforhodamine 101), a fluorescent chromophore group whose which wavelength is different from the fluorescently labeled L-glucose derivative described herein(maximum fluorescence wavelength thereof is around 580 nm to 630 nm), and examples thereof can contain 2-amino-2-deoxy-L-glucose. For example, 2-TRLG is a L-glucose derivative having sulforhodamine 101 at the 2-position by way of sulfonamide bond. Here, L-glucose derivative includes L-glucose itself, and additionally, L-glucose in which a hydroxyl group is substituted by an amino group, L-glucose in which a hydroxyl group is substituted by an amino group and in which a hydroxyl group is substituted by a fluorine atom, L-glucose having an amino group via a spacer, and L-glucose in which a hydroxyl group is substituted by a fluorine atom, and further, for example, molecules obtained by introducing fluorine at the 2-position, 3-position, 4-position, 6-position of L-glucose are also envisaged, and examples thereof include 2-F-6-TRLG, 3-F-2-TRLG, 4-F-2-TRLG and 6-F-2-TRLG. The derivative emitting red fluorescence is preferably 2-TRLG or sulforhodamine B, further preferably 2-TRLG.

It is reported that 2-TRLG is taken up into a cell having degraded plasma membrane condition by the present inventors (patent document 2). That is, since 2-TRLG is an L-form derivative, cellular uptake via binding to a glucose transporter does not occur, further, since 2-TRLG has a bulky and highly lipophilic fluorescent group, it is supposed, in the case when 2-TRLG enters into a cell, that the plasma membrane mechanism receives a relatively large damage, excluding a case by endocytosis. The larger the extent of the damage is, the faster the elimination of 2-TRLG from the cell is seen by washout. When a fluorescent reagent for determination of cell death (necrosis) such as widely used propidium iodide (PI) enters into a cell due to the plasma membrane damage, the reagent binds irreversibly to a nucleus, thereby showing the damage, however, it is difficult to express the degree of the damage. Unlike a molecule of which transmembrane permeation is inhibited simply by steric bulkiness such as dextran endowed with a fluorescent group, when 2-TRLG enters into a cell in which the degree of degradation of plasma membrane is not so remarkable as to presume cell death (necrosis) with complete destruction of plasma membrane but activity thereof still remain, it is adsorbed to the inner side of the plasma membrane and stays in the cell for a longer period of time. Therefore, when the fluorescently labeled L-glucose derivative described herein and 2-TRLG are used in combination, whether cells are cancer cells or not can be determined more correctly than in a case of solely using the L-glucose derivative, by bringing the fluorescently labeled L-glucose derivative and 2-TRLG detected at different fluorescence wavelengths into contact with the cells and by measuring the change in the color tone of the imaged cells depending upon the elapsed time as an index.

The fluorescently labeled L-glucose derivative, 2-NBDLG for example, and a fluorescently labeled L-glucose derivative, 2-TRLG for example, will be illustrated. The latter is taken up into tumor cells having somewhat degraded plasma membrane condition in a developed tumor cell cluster, whereas 2-NBDLG is taken up also into cells having utterly no degraded plasma membrane condition in a developed tumor cell cluster, in addition to the uptake into the above-described tumor cells. A fact that 2-NBDLG is taken up into these tumor cells as well is apparent also from Example 4. In contrast, when 2-TRLG is once taken up into a cell, it is not eliminated easily as compared with 2-NBDLG. Therefore, for example, if a solution containing 2-NBDLG emitting green fluorescence and 2-TRLG emitting red fluorescence mixed at a constant ratio (5:1) is prepared, and the sensitivities of green and red detectors are adjusted so that the proportion of the fluorescence intensities of these compounds is approximately 1:1 by irradiating the solution with suitable excitation light (488 nm), and if the above solution is allowed to be taken up into cells under such condition and the cells are imaged, then, the cells taking up both these compounds are visualized by yellow fluorescence as a result of superimposing green fluorescence derived from 2-NBDLG and red fluorescence derived from 2-TRLG. Thereafter, if 2-NBDLG is eliminated, 2-TRLG having a property of poor elimination would remain in the cytosol, and as a result the cells are visualized with red fluorescence. Therefore, in case when a change in the fluorescent color tone of cells from yellow to red along with the elapsed time is confirmed, it is understood that the cells once took up 2-NBDLG and 2-TRLG, and then, eliminated 2-NBDLG. According to WO2010/016587 regarding the prior study results of the present inventors, 2-TRLG are present as two isomer forms (ortho and para forms).

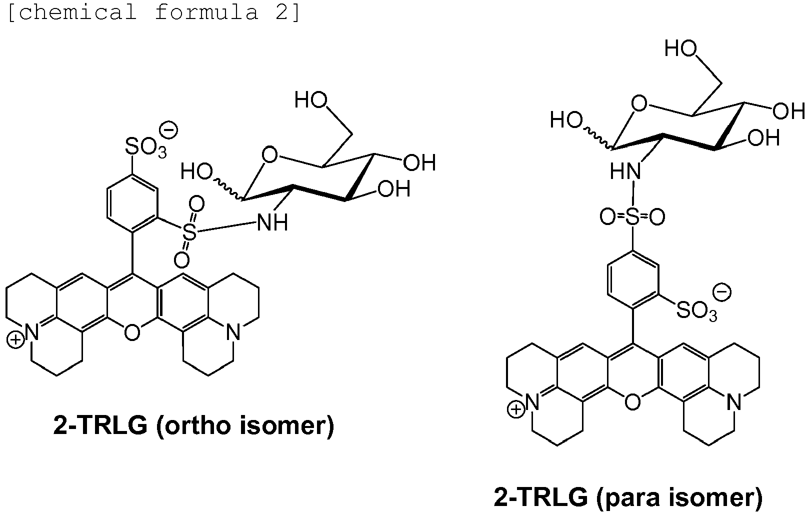

As described above, tumor cells constituting a developed tumor cell cluster take up 2-NBDLG and/or 2-TRLG, however, tumor cells taking up no 2-TRLG but taking up 2-NBDLG are present in the same tumor cell cluster and uptake of 2-NBDLG by these cells is inhibited by a substance inhibiting a specific transport pathway as shown in examples below. That is, the uptake of 2-NBDLG by these tumor cells is an uptake into tumor cells via a specific mechanism (here, the specific mechanism is a mechanism via a membrane protein through which labeled glucose derivatives showing a mutually enantiomeric relation, specifically, both 2-NBDLG and 2-NBDG can permeate, excluding permeation owing to breakdown of a lipid bilayer structure of plasma membrane as typified by permeation of a membrane impermeable marker such as propidium iodide), differing from non-specific uptake of 2-TRLG due to degradation of plasma membrane condition as disclosed in WO2010/016587. Therefore, by detecting uptake of 2-NBDLG as the fluorescently labeled L-glucose derivative into cells, tumor cells constituting a developed tumor cell cluster can be detected, namely, cancer cells can be detected.

2-TRLG is taken up into a cell exhibiting degraded plasma membrane condition. That is, it is taken up not only into tumor cells exhibiting somewhat degraded plasma membrane condition in the above-described developed tumor cell cluster, but also into cells damaged by inflammation or physical influences (including normal cells). Therefore, by using, for example, 2-TRLG in addition to the fluorescently labeled L-glucose derivative described herein, whether detection of the fluorescently labeled L-glucose derivative in target cells is due to the plasma membrane damage of the target cell or not can be evaluated, and as such, cancer cells can be detected with a high accuracy.

The fluorescently labeled L-glucose derivative described herein can further be radio-labeled. Specific examples thereof include 6-[¹⁸F] -2-NBDLG and 4-[¹⁸F] -2-NBDLG. By using an L-glucose derivative which is radio-labeled as well as fluorescently labeled, it becomes possible to detect cancer cells at the cellular level by confirming the site of cancer cells with whole body imaging using PET and subsequently detecting fluorescence, after administering the imaging agent containing the fluorescently labeled L-glucose derivative to a living body.

The fluorescently labeled L-glucose derivative described herein can also be used further in combination with a fluorescently labeled D-glucose derivative. Tumor cells have a nature of taking up D-glucose into the cell in larger amount as compared with normal cells (for example, non-patent document 16). Therefore, evaluation with higher accuracy is made possible by detecting the degree of uptake of D-glucose, in addition to detection of cancer cells using the fluorescently labeled L-glucose derivative.

The fluorescently labeled D-glucose derivative used in the case when combining detection using the fluorescently labeled L-glucose derivative and evaluation using the fluorescently labeled D-glucose derivative is not particularly restricted, providing that it is a D-glucose derivative which is taken up into a cell, and mentioned examples are D-glucose derivatives having a green or blue fluorescent chromophore group in the molecule, and specifically D-glucose derivatives having a 7-nitrobenz-2-oxa-1,3-diazole group in the molecule. Such D-glucose derivatives include, preferably, 2-[N-(7-nitrobenz-2-oxa-1,3-diazole-4-yl)amino]-2-deoxy-D-glucose (2-NBDG).

As apparent from the above-described explanations, the fluorescently labeled L-glucose derivative used in the present invention is useful for detecting cancer cells, and also useful, for example, as an active constituent of an imaging agent for visualizing cancer cells. The fluorescently labeled L-glucose derivative may be dissolved in a solvent (physiological saline for injection) for dissolving this and provided in the form of a solution, or may be combined with a solvent for dissolving this and provided in the form of a kit by which the derivative is dissolved to prepare a solution in use. The concentration of the labeled L-glucose derivative in a solution may be prepared, for example, in the range of 1 nM to 100 mM. It may also be permissible to further improve accuracy of the evaluation by combining the method of using the labeled L-glucose derivative described herein for detection of cancer cells with a method known per se.

### EXAMPLES

The present invention will be illustrated in detail by examples below, but the present invention is not construed to be limited to the following descriptions.

### Example 1: Synthesis of compound

### (1) Synthesis of fluorescently labeled L-glucose derivative

### (1-1) Synthesis of 2-NBDLG

2-NBDLG was synthesized from L-glucose as described below.

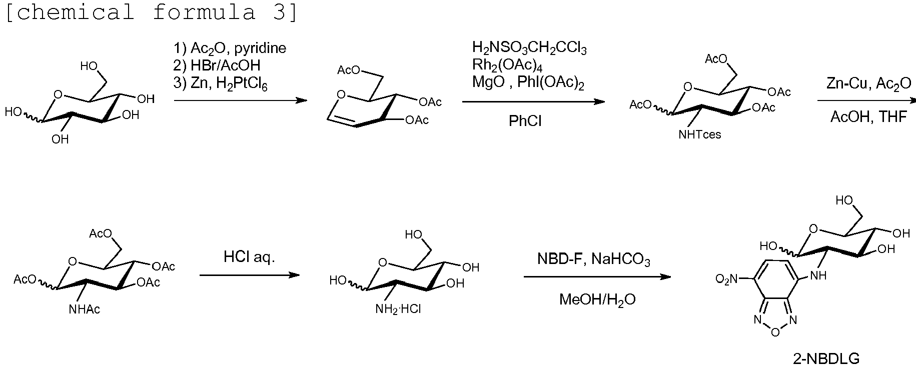

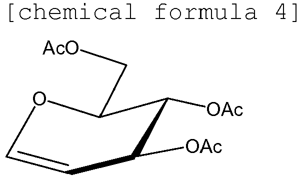

### 3,4,6-Tri-O-acetyl-L-glucal

L-glucose (20 g) was dissolved in pyridine (262 ml) and the solution was cooled to 0°C. Acetic anhydride (171 ml) was dropped, and the mixture was stirred overnight at room temperature. After concentration under reduced pressure, a process of performing azeotropy with toluene was repeated 3 times. To the residue was added ethyl acetate, and the mixture was washed with saturated sodium bicarbonate water and saturated saline, and the organic layer was dried over sodium sulfate. Sodium sulfate was filtered off, and the organic solvent was removed by concentration under reduced pressure. The resultant residue was dissolved in dehydrated dichloromethane (115 ml) under an argon atmosphere, and the solution was cooled to 0°C. A 30% hydrogen bromide acetic acid solution (61 ml) was dropped. After completion of dropping, the mixture was warmed up to room temperature and stirred for 3 hours. After 3 hours, the organic solvent was removed by concentration under reduced pressure. To the residue was added chloroform (800 ml), and the mixture was washed with saturated sodium bicarbonate water and saturated saline. The organic layer was dried over sodium sulfate. Sodium sulfate was filtered off. The organic solvent was removed by concentration under reduced pressure. To the resultant oily compound were added acetic acid (72 ml) and water (72 ml) and the mixture was cooled to 0°C. Zinc (71 g) was added bit by bit, and hexachloroplatinic (IV) acid (135 mg) was added. Further, acetic acid (72 ml) and water (72 ml) were added. After 1.5 hours, chloroform was added, and the mixture was filtrated through celite. To the filtrate was added saturated sodium bicarbonate water, and the organic layer was washed with saturated sodium bicarbonate water and saturated saline. The organic layer was dried over magnesium sulfate. Magnesium sulfate was filtered off, and the organic solvent was removed by concentration under reduced pressure. This was purified by silica gel column chromatography. The intended fractions were collected, and concentrated under reduced pressure to obtain an oily compound.
Yielded amount: 21.0 g
Yielded: 70% (in three steps)

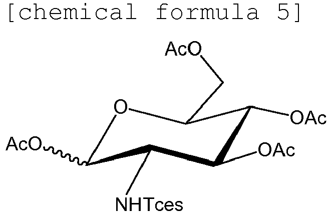

### 1,3,4,6-Tetra-0-acetyl-2-deoxy-2-(2',2',2'-trichloroethoxy sulfonylamino)-L-qlucopyranose

Chlorobenzene (73 ml) was added to 3, 4,6-Tri-O-acetyl-L-glucal (20 g), 2, 2, 2-Trichloroethoxysulfonylamine (18.5 g) and magnesium oxide (6.8 g) under an argon atmosphere, and rhodium acetate (650 mg) was further added. The mixture was cooled down to -20°C, and iodobenzene diacetate (30.8 g) was added. The temperature was returned to room temperature over a period of 2 hours, and the mixture was stirred overnight. It was diluted with chloroform, and the solution was filtrated through celite. The filtrate was concentrated under reduced pressure to obtain a brown oily compound which was then purified by silica gel column chromatography. The intended fractions were collected, to obtain a white solid.
Yielded amount: 26.0 g
Yielded: 63 %

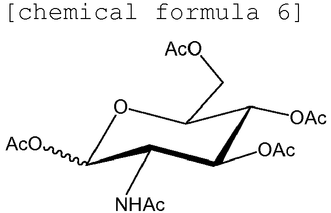

### 1,3,4,6-Tetra-O-acetyl-2-acetamido-2-deoxy-L-glucopyranose

1,3,4,6-Tetra-O-acetyl-2-deoxy-2-(2',2',2'-trichloroethoxysulfonylamino)-L-glucopyranose (22.9 g) was dissolved in acetic acid (130 ml) and acetic anhydride (130 ml) under an argon atmosphere. At room temperature, zinc-copper couple (40 g) was added and the mixture was stirred for 2 hours. The mixture was concentrated under reduced pressure, to the resultant residue was added chloroform, and the mixture was washed with saturated sodium bicarbonate water and saturated saline. The organic layer was dried over magnesium sulfate, and magnesium sulfate was filtered off. This was concentrated under reduced pressure to obtain the residue which was then purified by silica gel column chromatography. The intended fractions were collected, to obtain a white solid.
Yielded amount: 12.1 g
Yielded: 76 %

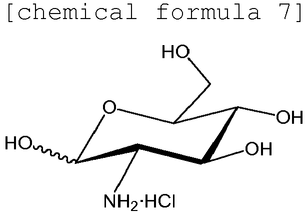

### L-glucosamine hydrochloride

1,3,4,6-Tetra-O-acetyl-2-acetamido-2-deoxy-L-glucopyranose (3.5 g) was suspended in 6M-HCL, and the suspension was heated at 70°C. After 6 hours, the suspension was concentrated under reduced pressure, and to the residue was added water and the mixture was subjected to azeotropy. The residue was freeze-dried from water, to obtain a white solid.
Yielded amount: 1.95 g
Yielded: 100 %

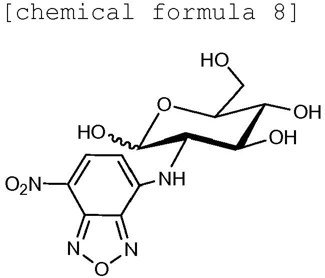

### 2-[N-(7-Nitrobenz-2-oxa-1,3-diazol-4-yl)amino]-2-deoxy-L-glucose (2-NBDLG)

NBD-F (227 mg) was charged in a brown flask, and dissolved in methanol (10 ml). Under an argon atmosphere, a solution prepared by dissolving L-glucosamine hydrochloride (100 mg) and sodium hydrogen carbonate (65.7 mg) in water (2.0 ml) was added, and the mixture was stirred at 37°C. After 2 hours, the organic solvent was removed by concentration under reduced pressure, and the generated precipitate (undesired substance derived from NBD-F) was washed with water. The filtrate and the wash solution were combined and purified by HPLC. The intended fractions were collected and freeze-dried.
Yielded amount: 125 mg
Yielded: 79 %
¹H-NMR (400 MHz, deuterated water, ppm): δ8.52 (d, 1H, J=9.1 Hz, H6'), δ6.56 and δ6.54 (d x 2, 0.5H x 2, J=9.1 Hz and J=9.1 Hz, H5'), δ5.38 (d, 0.5H, J=2.8 Hz, H-1α), δ4.89 (d, 0.5H, J=8.1 Hz, H-1β), δ3.50-δ4.02 (m, 6H, H-2, H-3, H-4, H-5, H-6, H-6).
ESI-MS: calcd for C₁₂H₁₅N₄O₈ [M+H]⁺ 343.1, found 343.1

### (1-2) Synthesis of 2-DBDLG

2-DBDLG was synthesized according to a method described in non-patent document 5, as described below.

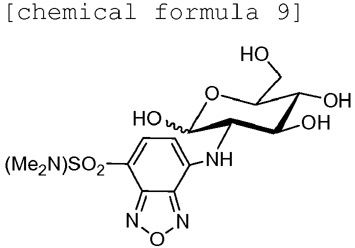

### 2-Deoxy-2-[N-7-(N',N'-dimethylaminosulfonyl)benz-2-oxa-1,3-diazol-4-yl)amino]-L-glucose (2-DBDLG)

DBD-F (100 mg) was charged in a brown flask, and dissolved in a mixed solvent composed of methanol (2.00 ml) and THF (2.00 ml). Under an argon atmosphere, a solution prepared by dissolving L-GlcNH₂.HCl (49.8 mg) in 0.3 M NaHCO₃ water (1.08 ml) was added, and the mixture was further washed thoroughly with water (1.00 ml), then, stirred at room temperature. After 48 hours, organic solvent was removed by concentration under reduced pressure, and the generated precipitate (undesired substance derived from DBD-F) was washed with water. The filtrate and the wash solution were combined and purified by HPLC. The intended fractions were collected and freeze-dried.
Yielded amount: 35.8 mg
Yielded: 38 %
¹H-NMR (400 MHz, deuterated water, ppm): δ7.82 and δ7.84 (d x 2, 0.5H x 2, J=8.3 Hz and 8.3 Hz, H-6' of DBD), δ6.42 and δ6.46 (d x 2, 0.5H x 2, J=8.3 Hz and 8.3 Hz, H-5' of DBD), δ5.31 (br.d, 0.5H, J=2.7 Hz, H-1α), δ4.79 (br.d, 0.5H, J=8.3 Hz, H-1β), δ3.43-δ3.90 (m, 6H, H-2, H-3, H-4, H-5, H-6, H-6), δ2.65 (s, 6H, Me₂NSO₂-).
ESI-MS: calcd for C₁₄H₂₁N₄O₈S [M+H]⁺ 405.1, found 405.1 Maximum fluorescence wavelength: 570 nm

### (1-3) Synthesis of 2,6-Dideoxy-6-[F]fluoro-2-[N-(7-nitrobenz-2-oxa-1,3-diazol-4-yl)amino]-L-glucose (6-F-2-NBDLG)

The structural formula is shown below.

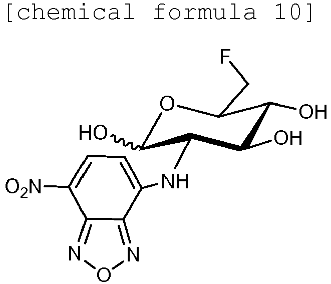

Another fluorescently labeled L-glucose derivative of the present invention, 6-F-2-NBDLG, was synthesized as described below (Synthesis method 1).

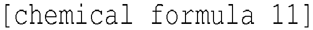

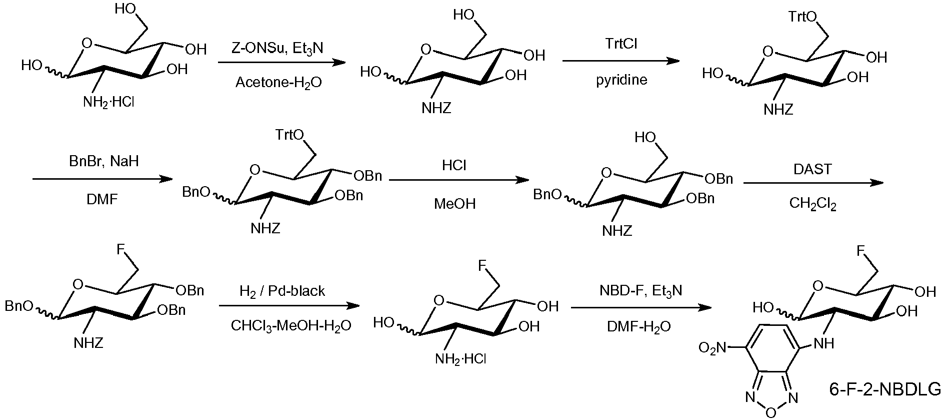

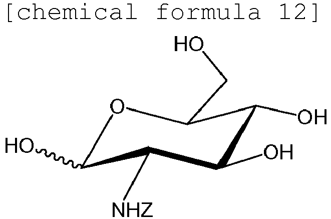

### 2-Benzyloxycarbonylamino-2-deoxy-L-glucopyranose

L-glucosamine hydrochloride (1.65 g) was dissolved in water (25 ml) and acetone (25 ml). Z-ONSu (2.1 g), acetone (25 ml) and triethylamine (2.8 ml) were added in series, and the mixture was stirred at room temperature. After 4 hours, the organic solvent and water were removed by concentration under reduced pressure. The generated precipitate (intended substance) was isolated by filtration, washed with water, then, dried under reduced pressure, to obtain a white solid.
Yielded amount: 1.75 g
Yielded: 73 %

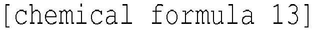

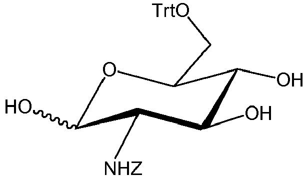

### 2-Benzyloxycarbonylamino-2-deoxy-6-0-triphenylmethyl-L-glucopyranose

2-benzyloxycarbonylamino-2-deoxy-L-glucopyranose (1.35 g) was dissolved in pyridine (80 ml) under an argon atmosphere, and trityl chloride (5.92 g) was added and the mixture was heated at 70°C and stirred. After 2 hours, it was left to cool, the organic solvent was removed by concentration under reduced pressure, and azeotropy with toluene was performed. The resultant residue was purified by silica gel column chromatography, and the intended fractions were collected, and concentrated under reduced pressure, to obtain a white solid.
Yielded amount: 2.2 g
Yielded: 92 %

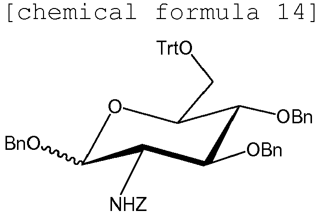

### Benzyl 3,4-di-O-benzyl-2-benzyloxycarbonylamino-2-deoxy-6-O-triphenylmethyl-L-glucopyranoside

2-benzyloxycarbonylamino-2-deoxy-6-O-triphenylmethyl-L-glucopyranose (2.1 g) was dissolved in dimethylformamide (36 ml) under an argon atmosphere, and benzyl bromide (713 µl) was added and the mixture was stirred. The reaction mixture was cooled to 0°C, and sodium hydride (content: 60%, 433 mg) was added and the mixture was stirred. After 1 hour, sodium hydride (content: 60%, 43 mg) was additionally added. After 2 hours, ice was added, then, the mixture was extracted with ethyl acetate, and washed with saturated saline. The organic layer was dried over sodium sulfate, and sodium sulfate was filtered off. This was concentrated under reduced pressure to obtain the residue which was then purified by silica gel column chromatography. The intended fractions were collected, and concentrated under reduced pressure, to obtain an oily compound.
Yielded amount: 2.0 g
Yielded: 64 %

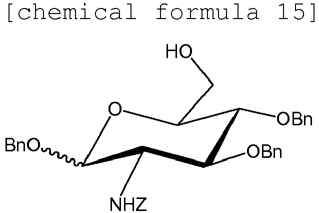

### Benzyl 3,4-di-O-benzyl-2-benzyloxycarbonylamino-2-deoxy-L-glucopyranoside

Benzyl-3,4-di-O-benzyl-2-benzyloxycarbonylamino-2-deoxy-6-O-triphenylmethyl-L-glucopyranoside (2.0 g) was dissolved in methanol (40 ml) and chloroform (4 ml). A 4.4 M hydrogen chloride-dioxane solution (8 ml) was added and the mixture was stirred. After 2 hours, this was concentrated under reduced pressure to obtain the residue which was then purified by silica gel column chromatography. The intended fractions were collected and concentrated under reduced pressure, to obtain a white solid.
Yielded amount: 650 mg
Yielded: 46 %

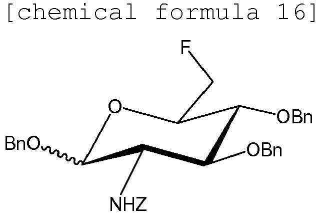

### Benzyl 3,4-di-O-benzyl-2-benzyloxycarbonylamino-2,6-dideoxy-6-fluoro-L-glucopyranoside

Benzyl-3,4-di-O-benzyl-2-benzyloxycarbonylamino-2-deoxy-L-glucopyranoside (95 mg) was dissolved in dichloromethane (1.6 ml)under an argon atmosphere and the solution was stirred, and cooled to -20°C. N,N-diethylaminosulfur trifluoride (107µl) was added and the mixture was stirred and warmed up to room temperature. After 2 hours, methanol was added, and the mixture was extracted with saturated sodium bicarbonate water and ethyl acetate. The organic layers were combined and washed with saturated sodium bicarbonate water and saturated saline. The organic layer was dried over sodium sulfate, and sodium sulfate was filtered off. This was concentrated under reduced pressure to obtain the residue which was then purified by silica gel column chromatography. The intended fractions were collected, and concentrated under reduced pressure, to obtain a white solid.
Yielded amount: 63 mg
Yielded: 66 %

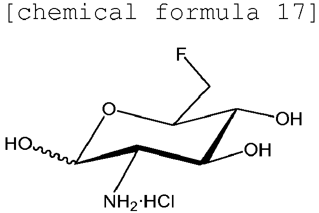

### 2-Amino-2,6-dideoxy-6-fluoro-L-glucopyranose hydrochloride

Benzyl-3,4-di-O-benzyl-2-benzyloxycarbonylamino-2,6-dideoxy-6-fluoro-L-glucopyranoside (62 mg) was dissolved in chloroform/methanol/1M HCl = 3/6/1 (5 ml) and the solution was stirred. Palladium-black (40 mg) was added and the mixture was stirred, and substituted by hydrogen. After 8 days, palladium-black was filtered off, and an oily compound was obtained by concentration under reduced pressure.
Yielded amount: 23.1 mg
Yielded: 100 %

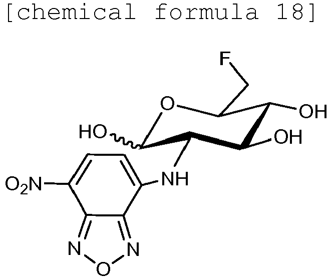

### 2,6-Dideoxy-6-fluoro-2-[N-(7-nitrobenz-2-oxa-1,3-diazol-4-yl)amino]-L-glucopyranose (6-F-2-NBDLG)

2-Amino-2,6-dideoxy-6-fluoro-L-glucopyranose hydrochloride (23.1 mg) was dissolved in dimethylformamide/water = 10/1 (2 ml) and the solution was stirred. NBD-F (23.2 mg) was added, further, triethylamine (32.4µl) was added and the mixture was warmed to 37°C. After 2 hours, acetic acid was added for neutralization, and water was added and the mixture was allowed to pass through a membrane filter. The filtrate and the wash solution were combined and purified by HPLC. The intended fractions were collected and freeze-dried, to obtain a red solid.
Yielded amount: 11.5 mg
Yielded: 32 %
¹H-NMR (400 MHz, deuterated water, ppm) : δ8.43 (d, 1H, J=8.9 Hz, H6'), δ6.49 and δ6.45 (d x 2, 0.5H x 2, J=9.2 Hz and J=9.2 Hz, H5'), δ5.33 (d, 0.5H, J=2.3 Hz, H-1α), δ4.89 (d, 0.5H, J=7.8 Hz, H-1β), δ4.48-δ4.63 (m, 1H, H-2), δ3.90-δ4.00 (m, 1H, H-3), δ3.52-δ3.71 (m, 3H, H-4, H-5, H-6). ESI-MS: calcd for C₁₂H₁₄FN₄O₇ [M+H]⁺ 345.1, found 345.0 Maximum excited wavelength: 472 nm
Maximum fluorescence wavelength: 538 nm

6-F-2-NBDLG can be synthesized also as described below (Synthesis method 2).

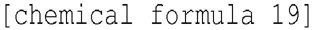

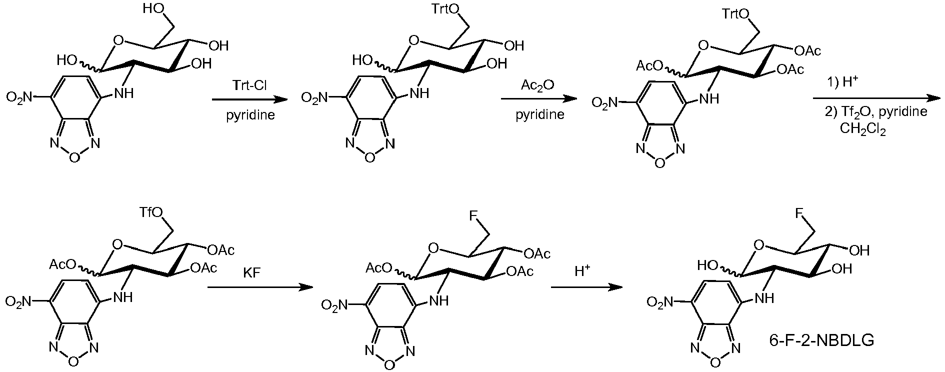

Here, another fluorescently and radiolabeled L-glucose derivative of the present invention, 6-¹⁸F-2-NBDLG, can be synthesized by using K¹⁸F instead of KF.

### Reference Example 1: Synthesis of comparative compound

### (1) Synthesis of fluorescently labeled D-glucose derivative

### (1-1) Synthesis of 2-NBDG

2-NBDG was synthesized according to a method described in non-patent document 5.

### (1-2) Synthesis of fluorescently labeled L-mannose

In the present invention, L-glucose which is an enantiomer of D-glucose as a native form hexose present in a large amount in the natural world and is non-native form not found in the natural world was used as the sugar skeleton to which a fluorescent group is linked. On the other hand, the present inventors have newly synthesized 2-Deoxy-2- [N- (7-nitrobenz-2-oxa-1,3-diazol-4-yl) amino]-L-mannose (2-NBDLM), as a fluorescently labeled L-mannose derivative having a skeleton composed of L-mannose which is an enantiomer of D-mannose as a hexose present in a large amount in the natural world and is non-native form.

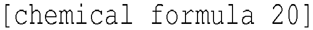

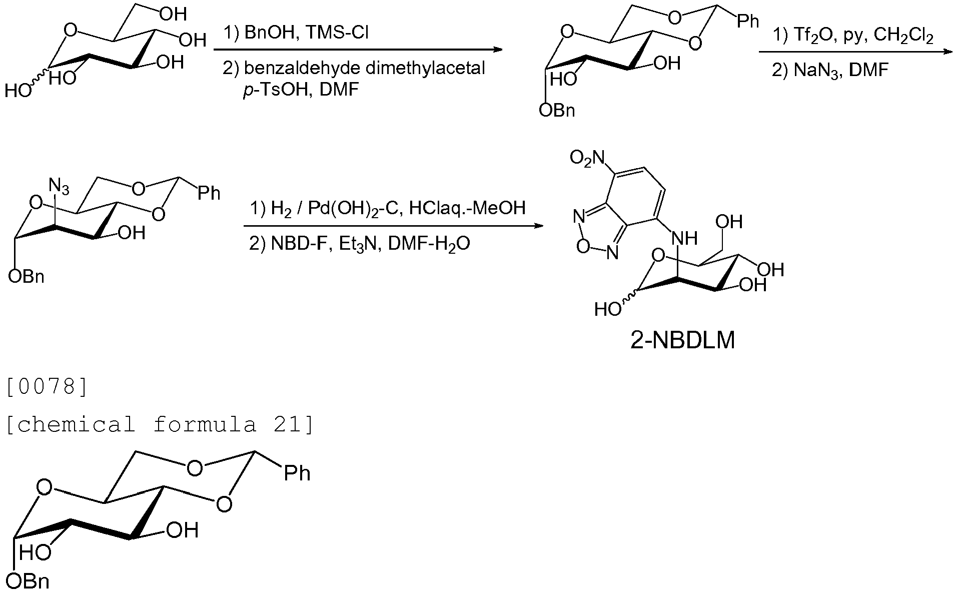

### Benzyl 4,6-O-benzylidene-α-L-glucopyranoside

L-glucose (6 g) was suspended in benzyl alcohol (173 ml) . At room temperature, chlorotrimethylsilane (42.3 ml) was dropped and the mixture as stirred at 60°C. After 5 hours, to the reaction solution were added ether and water and extraction thereof was performed. The resultant aqueous layer was washed with ether, and the aqueous layer was freeze-dried. The resultant solid was dissolved in dimethylformamide (150 ml) . Under an argon atmosphere, p-toluenesulfonic acid monohydrate (633 mg) and benzaldehyde dimethyl acetal (7.5 ml) were added in series and the mixture was stirred at 70°C. After stirring for 3 hours, benzaldehyde dimethyl acetal (2.5 ml) was additionally added. The mixture was further stirred for 6 hours, then, the temperature was returned to room temperature and the mixture was stirred overnight. After stirring overnight, to the reaction solution were added ethyl acetate and saturated sodium bicarbonate water and extraction thereof was performed. The resultant organic layer was washed with saturated saline, then, dried over anhydrous magnesium sulfate, and filtered off, then, concentrated under reduced pressure. The resultant solid was recrystallized from ethanol.
Yielded amount: 2.50 g
Yielded: 21% (in two steps)

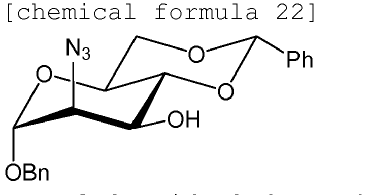

### Benzyl 2-azido-2-deoxv-4,6-O-benzylidene -α-L-mannopyranoside

Pyridine (449 µl) was dissolved in dichloromethane (150 ml). Under an argon atmosphere, trifluoromethanesulfonic anhydride (257 µl) was dropped at -30°C. After 10 minutes, benzyl 4,6-O-benzylidene-α-L-glucopyranoside (500 mg) was added and the mixture was stirred at -30°C. After 2 hours, to the reaction solution were added chloroform and saturated saline and extraction thereof was performed. The resultant organic layer was dried over anhydrous sodium sulfate, filtered off, then, concentrated under reduced pressure. The resultant oily compound was dissolved in dimethylformamide (10 ml), and under an argon atmosphere, sodium azide (272 mg) was added and the mixture was stirred at 50°C. After 3 hours, sodium azide (635 mg) was additionally added and the mixture was stirred at room temperature. Further after 46 hours, sodium azide (906 mg) was additionally added and the mixture was stirred at room temperature. After 20 hours, to the reaction solution were added ether and water and extraction thereof was performed. The resultant organic layer was washed with water, then, dried over anhydrous magnesium sulfate, filtered off, then, concentrated under reduced pressure. The resultant residue was purified by silica gel column chromatography, to obtain the intended substance.
Yielded amount: 172 mg
Yielded: 32%

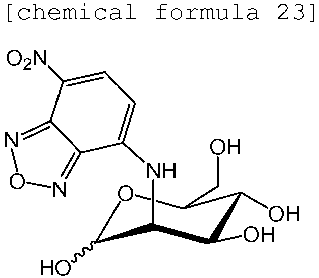

### 2-Deoxy-2-[N-(7-nitrobenz-2-oxa-1,3-diazol-4-yl)amino]-L-mannose (2-NBDLM)

Benzyl-2-azido-2-deoxy-4,6-O-benzylidene-α-L-manno-pyranoside (172 mg) was dissolved in methanol (5 ml) and 2M HCl water (449 µl). Under an argon atmosphere, 10% Pd (OH)₂/C (43 mg) was added, hydrogen was added and the mixture was stirred. After 20 hours, 10% Pd(OH)₂/C was filtered off, and concentrated under reduced pressure. The resultant oily compound was dissolved with water, then, washed with ether, and the aqueous layer was concentrated under reduced pressure. The resultant oily compound was dissolved in dimethylformamide (2 ml) and water (387 µl). At room temperature, NBD-F (82 mg) and triethylamine (124 µl) were added in this order and the mixture was stirred. After 1.5 hours, the mixture was quenched with acetic acid (80 µl), and purified by HPLC. The intended fractions were collected and freeze-dried.
Yielded amount: 37 mg
Yielded: 24%
¹H-NMR (400 MHz, deuterated water, ppm): δ8.44 (d, 1H, J=9.1 Hz, H6'), δ6.55 and δ6.51 (d x 2, 0.5H x 2, J=9.1 Hz and J=9.1 Hz, H5'), δ5.24 and δ5.09 (0.5H x 2, H-1), δ3.35-δ4.14 (m, 6H, H-2, H-3, H-4, H-5, H-6, H-6).
ESI-MS: calcd for C₁₂H₁₅N₄O₈ [M+H]⁺ 343.1, found 343.0 Maximum excited wavelength: 472 nm
Maximum fluorescence wavelength: 539 nm

### Example 2: Investigation using mouse transplanted with tumor cells (C6 glioma)

### (Experimental methodology)

### (1) Preparation of rat glioma cells (C6) for transplantation

C6 cells were prepared according to an ordinary method so that the cell number was 2 x 10⁷ cells/mL.

### (1-1) Culture of C6 cells

C6 cells were seeded on a 10 cm petri dish, allowed to stand still in a CO₂ incubator, and cultured at 37°C. The culture medium was exchanged once every two days.

### (1-2) Composition of the culture medium used for culture of C6 cells

1 g/L glucose-containing Dulbecco's modified Eagle's Medium (DMEM) (Nacalai No. 08456-65), to which Fetal Bovine Serum (Equitech-Bio SFBM) was added so that the final concentration was 10 % and penicillin-streptomycin (Nacalai No. 26253-84) was added so that the final concentration was 1 %, was used.

### (2) Preparation of tumor model mouse

5 to 6-week old athymic nude mice (BALB/cAJcl-nu/nu) were transplanted with 50 µL of a cell suspension adjusted to 2 x 10⁷ cells/mL subcutaneously around the root of right hind paw, under isoflurane anesthesia. The transplanted tumor cells were observed until growing to about 200 to 300 mm³, and used in vivo fluorescent imaging.

### (3) In vivo fluorescent imaging using C6 tumor model mice

C6 tumor model mice were allowed to stand still in an in vivo fluorescence imaging apparatus IVIS (registered trademark) Kinetics (Caliper Life sciences) under isoflurane anesthesia and autofluorescence of the mouse skin was measured, and subsequently, 400 nmol (100 µL) of 2-NBDLG or 2-NBDG dissolved in physiological saline was administered from the tail vein over a period of 30 seconds, and measurement of the fluorescence imaging was started again from one minute after starting administration. The in vivo fluorescence imaging was measured at an excitation wavelength of 465 nm using a GFP filter (transmittable fluorescence wavelength: 515-575 nm). The measurement was conducted for 45 minutes, and images were taken every one minute during the initial 20 minute and every 5 minutes from 20 to 45 minutes, obtaining 25 images in total. The accumulated values (fluorescence value) of 2-NBDLG and 2-NBDG in the interested area were obtained by subtracting the autofluorescence images from the 25 images taken after administration of 2-NBDLG or 2-NBDG.

### (3-1) Preparation of 2-NBDLG and 2-NBDG solution

A vial of 0.5 mg of 2-NBDLG or 2-NBDG was dissolved in 400 µL of physiological saline directly before the experiment, to prepare a solution having a final concentration of 400 nmol/100 µL.

### (Experimental result)

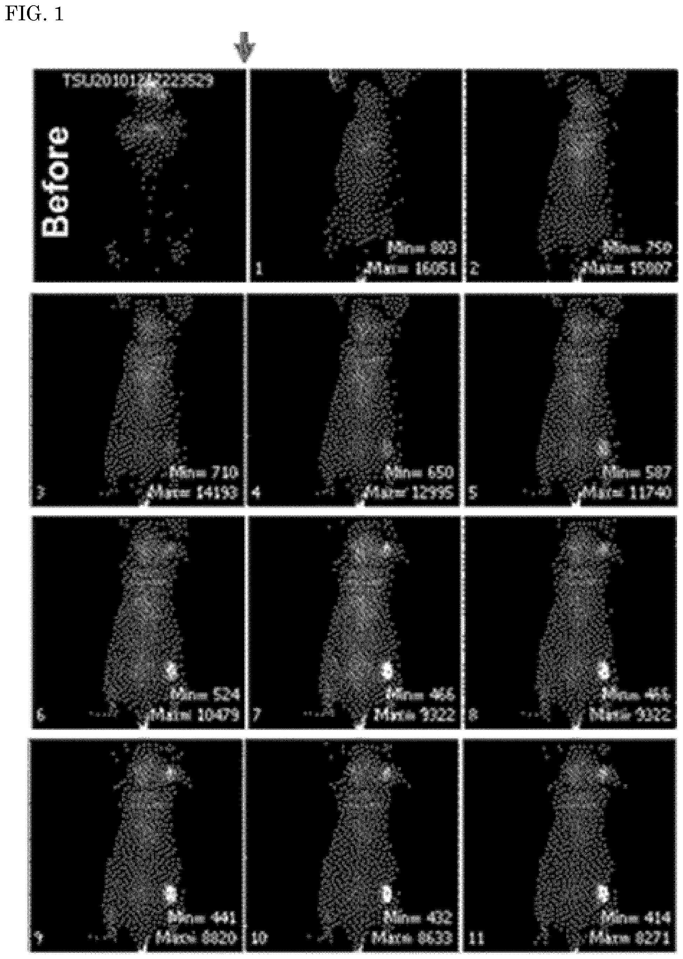

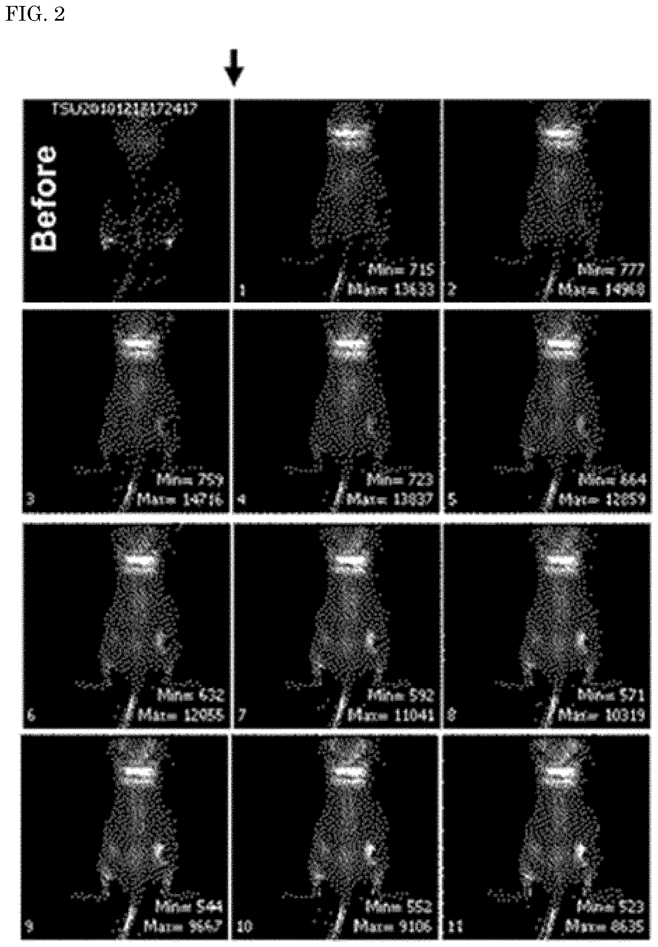

Fig. 1 shows fluorescence images acquired every one minute after administering 2-NBDLG to nude mice transplanted with tumor cells (C6 glioma) at the root part of right hind paw. The number denotes time after administration. 2-NBDLG was administered at a point of time indicated by an arrow at a concentration of 400 nmol/100 µL from the tail vein over a period of 30 seconds. Since 2-NBDLG shows intense accumulations at the tumor-transplanted part and is scarcely taken up into normal tissue, excellent contrast is obtained in a short period of time, as compared with the case of administration of 2-NBDG (Fig. 2) . Here, non-fasted mice were used. Fig. 2 shows fluorescence images acquired every one minute after administration of 2-NBDG under the same conditions as in administration of 2-NBDLG. Intense accumulations of 2-NBDG (indicated by yellow) are recognized at fat tissue on the back and between back bones, and at muscle, showing poor contrast from the tumor-transplanted part.

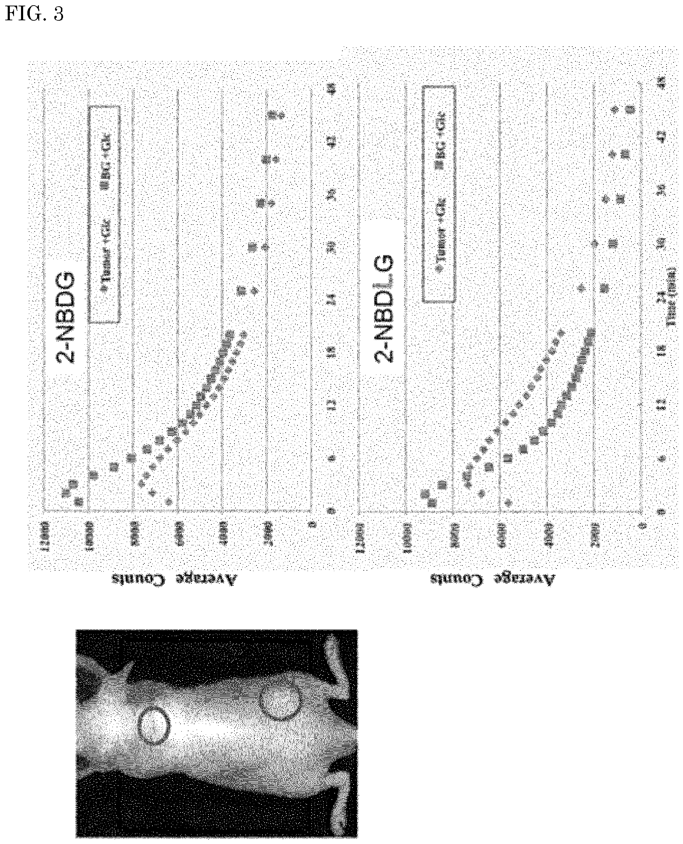

The temporal changes in the accumulated values of 2-NBDLG and 2-NBDG at the tumor cell-transplanted part (Tumor+Glc) around the root of right hind paw set as the region of interest and at a dorsal part (BG+Glc) rich in adipocyte near neck are shown in Fig. 3. As apparent from Fig. 2, in the case when 2-NBDG was administered, accumulations were recognized at the dorsal part rich in adipocyte near neck just after administration. Accumulations at the tumor cell-transplanted part were recognized from around 5 minutes after administration, however, the contrast from the adipocyte-rich dorsal part near neck was not sufficient. In contrast, in the case when 2-NBDLG was administered, specific accumulations at the tumor cell-transplanted part were recognized from around 5 minutes after administration, and high contrast from the adipocyte-rich dorsal part near neck was obtained, as apparent from Fig. 1. This result was supported by the results shown in Fig. 3 as well. That is, it is understood that in the example of administration of 2-NBDG, the fluorescence values at normal tissue (red square, BG) are larger than at the tumor-transplanted part (blue rhombus, Tumor), whereas in the example of administration of 2-NBDLG, the fluorescence values at the tumor-transplanted part (blue rhombus, Tumor) are consistently larger than at normal tissue (BG). The same result was obtained in 6 examples in total. From the above-described results, effectiveness of 2-NBDLG in evaluating whether cells are cancer cells or not became apparent.

### Example 3: Imaging of tumor cell cluster composed of mouse insulinoma cells (MIN6) using 2-NBDLG

### (Experimental methodology)

### (1) Preparation of mouse insulinoma cells (MIN6)

Culture medium containing MIN6 cells suspended at a proportion of 10 x 10⁴ cells/mL was dispersed by pipetting, and dropped on a glass cover slip, then, allowed to stand still in a 5%CO₂ incubator for 20 minutes to cause adhesion to the glass surface, and 3 mL of the culture medium was added, and the cells were cultured. The half quantity of the culture medium was exchanged every two days.

### (1-1) Culture of MIN6 cells

MIN6 cells (provided by Professor Miyazaki Junichi, Osaka University, and the passage number was six to nine times) were seeded on a 10 cm petri dish, allowed to stand still in a CO₂ incubator, and cultured at 37°C. The half quantity of the culture medium was exchanged every two days.

### (1-2) Composition of culture medium used for culture of MIN6 cells

High glucose-containing Dulbecco's modified Eagle's Medium (DMEM-HG) (SIGMA #D5648) (13.4 g), NaHCO₃ (Wako, No.191-01305) (3.4 g) and 2-Mercaptoethanol (Wako, No. 135-14352) (5 µL) were dissolved in 1 liter of ultra-pure water (Mili Q), and equilibrated with ambient air. Next, pH was adjusted to 7.3 to 7.35 using 1N HCl in a CO₂ incubator at 37°C, and sterilized by suction filtration. Directly before use, Hyclone Fetal Bovine Serum (Cat# SH30070.03) was added so as to give a final concentration of 10 % and penicillin-streptomycin (Gibco #15140) was added so as to give a final concentration of 0.5 %.

### (1-3)Culture medium containing MIN6 cells suspended at proportion of 10 x 10⁴ cells/mL

MIN6 cells were prepared with culture medium so that the cell number was 10 x 10⁴ cells/mL.

### (2) Preparation of 2-NBDLG solution

The whole quantity of one vial of 0.5 mg 2-NBDLG was dissolved in 14.6 mL of a HEPES solution for image acquisition, for preparing a 2-NBDLG solution having a final concentration of 100 µM.

### (2-1) HEPES solution for image acquisition

NaCl 131.8 mM, KCl 4.75 mM, KH₂ PO₄ 1.19 mM, MgCl₂ 1.2 mM, CaCl₂ 1 mM, NaHCO₃ 5.0 mM, D-Glucose 2.8 mM, HEPES 10 mM (adjusted to pH 7.35 with 1M NaOH). For inhibiting entrance and elimination of fluorescently labeled glucose via a gap junction/hemichannel, 0.1 mM Carbenoxolone (SIGMA #C4790) was added. The HEPES solution for image acquisition was used as a solution for preparing a 2-NBDLG solution and as a solution for preparing a 2-NBDLG/2-TRLG solution.

### (3) Administration of DAPI solution to MIN6 cells

A glass cover slip to which MIN6 cells had been adhered and wherein MIN6 cells had been cultured for 10 to 13 days was transferred into a DAPI solution containing 5.6 mM D-glucose filled in a 35 mm dish, and allowed to stand still for 45 minutes to 1 hour while warming at 37°C to allow cells to take up DAPI. In a separate experiment, DAPI was administered while continuously observing on a confocal microscope, and it was confirmed that the morphological change of the cell due to DAPI administration and irradiation with 405 nm laser was not recognized during the experimental period.

### (3-1) Preparation of DAPI solution

A solution prepared by dissolving 4',6-Diamidino-2-phenylindole DAPI(No. 049-18801, Wako Pure Chemical Industries, Osaka) at a concentration of 100 mg/mL in an aqueous solution containing 10 % DMSO was refrigerated, and in use, diluted 100 times with a HEPES solution containing 5.6 mM D-Glucose for DAPI preparation.

### (4) Method of fixing glass cover slip wherein MIN6 cells have been cultured into perfusion chamber for fluorescence measurement using metal guide

A glass cover slip wherein MIN6 cells had been cultured was transferred into a HEPES solution for image acquisition in a perfusion chamber set on a universal stage (Leica 11600234) on a confocal laser scanning microscope (TCS SP5 available from Leica), and slightly adhered closely to the glass surface at the bottom of the chamber. After allowing to stand still, the both side of the cover slip were held and carefully pressed by two rectangular metal guides (length: 10 mm, width: 2 mm, thickness: 0.7 mm, made of silver) in parallel to the long axis of the cover slip from the right and left sides thereof, so that the cover slip did not move even in the flow. Further, there is an excellent effect that in the space sandwiched by the metal guides, the perfusion solution flows smoothly as a laminar flow and quick liquid exchange is possible.

### (4-1) Perfusion chamber for fluorescence measurement on confocal laser scanning microscope stage

On an aluminous warming control platform having a round hole (diameter: 18 mm) at the bottom for an objective lens (PH1, Warner Instruments, USA, warmed at 37°C by a temperature control apparatus TC-324, Warner Instruments), a cover glass (width: 24 mm x length: 50 mm, thickness: No. 1, Warner Instruments, No. CS-24/50) was closely adhered to parts other than the round hole at the center of the platform using a silicon grease (HIVAC-G, Shin-Etsu Silicone, Tokyo). Then, on the cover glass, a silicon plate having a thickness of 1 mm (width: 20 mm x length: 50 mm) on which opening in the form of streamline had been performed at the center (at the side in contact with the glass bottom, width: 10 mm x length: 35 mm, curvature radius: 33 mm, and at the side not in contact with the glass surface, namely, at the upper side, the size is slightly wider) was placed, and adhered closely to the cover glass without using a silicon grease.

At the upstream corner of the streamline-shaped hole on the silicon plate, a 20 gauge Cattelan needle having a blunt tip was set and used as an inlet.

As a stainless tube for removing a perfusion solution (outlet), a tube having a tip crushed flatly and cut obliquely according to a method described in non-patent document 16 was used (if working is difficult, may be substituted by one obtained by linearly stretching a stainless outlet attached to plastic perfusion chamber such as RC-24N available from Warner), and in vacuum suction, both air and a solution were sucked simultaneously to attain stabilization.

### (5) System of feeding perfusion solution to perfusion chamber

### (a) Warming of perfusion solution and feeding thereof to perfusion chamber

A perfusion solution feeding system is equipped with one 60 mL cylinder for a control solution and five 10 mL cylinders for agent feeding, which can be switched as needed by a magnetic valve to allow perfusion. In experiments according to the present invention, a 2.8 mM glucose-containing HEPES solution for image acquisition was administered using the 60 mL cylinder and a mixed solution of 2-NBDLG and 2-TRLG was administered using one of the five 10 mL cylinders. As described below, to avoid generation of bubbles in the perfusion chamber, both the solutions were heated beforehand, combined in one tube before being introduced into the perfusion chamber, the flow rate thereof being controlled by a flow rate controller, then, heated again by an inline heater and fed to the perfusion chamber on the confocal microscope.

The HEPES solution for image acquisition was fed from the 60 mL cylinder warmed in an aluminum syringe heater (Model SW-61, temperature control unit is No. TC-324B, Warner Instruments) to a three-way stopcock for flushing the inside of a tube of a solution feeding line, subsequently, to the normally opened side of an ultra-compact magnetic valve (EXAK-3, 3 way clean valve, Takasago Electric, Nagoya) via a thin and lowered gas-permeability soft tube (PharMed tube, AY242409, Saint-Gobain Performance Plastics, Ohio). Opening and closing of the magnetic valve was controlled by a pulse generating apparatus (Master 8, manufactured by AMPI, Israel). The HEPES solution for image acquisition was fed continuously from a medium bottle into the 60 mL cylinder using a peristaltic pump (MCP pump, 12 rollers, Ismatec), and the solution feeding speed of the pump was controlled accurately to obtain the same value as the solution dropping speed so that the height of the upper surface of the solution in the cylinder did not change during the experiment. Since the solution feeding speed of the peristaltic pump is displayed digitally, if the speed of feeding the solution to the perfusion chamber changes during the experiment, it is immediately detected based on a change in the height of the solution surface. Since this solution is constantly renewed, a syringe heater SW-61 was set at 38.5°C for maintaining the liquid temperature.

On the other hand, the mixed solution of 2-NBDLG and 2-TRLG was fed from the 10 mL cylinder warmed at 37.5°C set in a syringe heater (Model SW-6, temperature control unit is No. TC-324, Warner Instruments) . The cylinder is connected to the normally closed side of a magnetic valve different from one for the HEPES solution for image acquisition, and switching to the control solution can be performed as needed by control of a pulse generating apparatus and the control solution can be fed. Six 10 mL cylinders can be set on the syringe heater SW-6, and distilled water was charged in one of them and a probe for monitoring the temperature of a heating block was inserted.

The HEPES solution for image acquisition as a control solution and the mixed solution of 2-NBDLG and 2-TRLG were, after going out of the outlet of the magnetic valve, collected in one route by a compact manifold (MPP-6, Warner Instruments) having 6 ports. The outlet of the MPP-6 manifold was connected to a short PharMed tube, and this tube was inserted into a flow rate controller which can increase and decrease the aperture by a screw, and the flow rate was regulated as 1.2 ± 0.2 mL/minute by controlling the aperture. This PharMed tube was connected to an inline heater (Multi-Line In-Line Solution Heater SHM-8, temperature control unit is TC-324B, Warner Instruments) in the shortest distance. It is because the temperature of the solution to be introduced into a perfusion chamber is warmed immediately before introduction. The temperature of the SHM-8 inline heater was so regulated that the actually measured temperature of a perfusion solution in the chamber was 36-37°C in the region where the cover slip exists, according to the perfusion speed. The warmed solution was connected to a stainless pipe (inlet) placed upstream of the perfusion chamber in the shortest distance via a short Tygon tube (R-3603, inner diameter 1/32 inch) and fed to the perfusion chamber.

Since pressure of feeding a solution from a cylinder is determined by using hydrostatic pressure, a difference in height may generates a difference in perfusion speed, to cause a variation in the height of the water surface in a chamber. To avoid this, for a 2-NBDLG solution, liquid feeding is not performed during an experiment in a single experiment, and after completion of each experiment, a solution was added so that the liquid upper surface showed approximately the same height with a no-fluorescent glucose-containing HEPES solution, because the administration time of the solution is short. Further, by carefully controlling the length and the thickness of a tube connected to a cylinder so as to cause flow at the same speed as the perfusion speed of a HEPES solution for image acquisition as a control solution, a variation of the liquid surface due to liquid exchange can be avoided. After completion of the experiment and before starting thereof, the inside of a tube was flashed sufficiently to ensure smooth flow.

### (b) Maintenance of laminar flow in perfusion chamber and removal of perfusion solution

Smooth and rapid removal of a perfusion solution from on a perfusion chamber is one of the most important points for obtaining a highly reproducible result. A stainless tube (outlet) for removing a perfusion solution was introduced to two large glass traps in series by a Tygon tube, and calmly sucked by a vacuum pump (DAP-15, ULVAC KIKO, Inc.). The suction pressure was monitored by a pressure gauge installed in a line branched from a suction line in the middle of two large glass traps, and adjusted to 35 kPa by controlling the degree of opening and closing of a three-way stopcock.

For maintenance of a laminar flow in a perfusion chamber, first, a solution of a blue dye (Pontamine sky blue, diluted to a concentration of 1% or less in use) was dropped around an inlet, and the left-right symmetry, uniformity and reproducibility of flow were ensured.

For confirmation of the temperature of each part in a perfusion solution in a chamber, an ultrafine thermistor probe (IT-23 manufactured by Physitemp) was used (non-patent document 16). The tip of an outlet was observed by an operation microscope (POM-50II, KONAN MEDICAL, Nishinomiya) installed on a chamber and cleaned in every experiment, for preventing a variation of suction pressure due to attachment of a salt derived from a HEPES solution during the experiment. This microscope is useful in installing a cover slip onto a chamber, and for confirmation of flow state, abnormalities in a chamber, in addition to the above-described action.

### (6) Image acquisition condition

A confocal laser scanning microscope (TCS-SP5 system manufactured by Leica, microscope body is DMI6000 CS trino electromotive inverted microscope) was used in conventional mode. Regarding laser used, DAPI used for nuclear staining was excited at an acoustic optical polarization element (Acoustic Optical Tunable Filter, AOTF) 20 % using 405 nm diode laser, and 2-NBDLG and 2-TRLG were excited at 488 nm by Argon laser main power 30-60% and AOTF 20-60%. The scanning speed used was 200Hz or 400Hz.

For fluorescence detection, a photomultiplier detector (PMT)1 was used in the wavelength detection range of 415-500 nm (detection sensitivity: 750 V) for detection of blue fluorescence by DAPI, and PMT2 (called green channel, the same shall apply hereinafter) was used in the wavelength detection range of 500-580 nm(detection sensitivity: 670 V) for detection of green fluorescence by 2-NBDLG. Selection of the above-described blue (415 to 478 nm) and green (500 to 580 nm) fluorescence detection wavelength ranges was carried out by a system combining a prism spectrum and a slit (standard of TCS-SP5, Leica), not depending on an emission filter system usually used. A beam splitter was used at 500 nm (RSP500). A beam splitter for 405 nm was of fixed at 415 nm in the SP5 system, independently of the above-described system. In this experiment, when exciting fluorescently, first, image acquisition along with time course of a 2-NBDLG administration protocol was carried out using excitation at 488 nm, and next, nuclear morphologies, which may vary depending on the depth in the z-axis direction, were obtained in an xyz mode by excitation at 405 nm.

In the acquisition of differential interference contrast (DIC) image for capturing the three-dimensional structural feature of a tumor cell cluster, one detected by a detector for transmitted light (PMT Trans, typical detection sensitivity: 145 to 200 V) simultaneously activated during excitation at 488 nm was used. For avoiding problems of the switching time and the switching shock when inserting a polarizer and an analyzer necessary for image acquisition of a differential interference mode (DIC) into an optical path, the polarizer and the analyzer for DIC were allowed to remain in the optical path even during the image acquisition by 405 nm excitation.

In this method, for obtaining high resolution for the xy axis and an angle of view to include the whole cell cluster in the field of view, an objective lens having high resolution, x40oil lens (HCX PL APO CS 40.0x1.25 OIL UV, NA1.25) was used with the aperture opened. For increasing the acquired fluorescence intensity, the pinhole size was set at 3 airy units. It was confirmed in the acquired image that nucleus and cytoplasm within the cell can be practically discriminated in the z-axis direction even with this pinhole size. Scanning was carried out twice for reducing noises, without using zoom (1x) at a number of pixels of 1024x1024, and the averaged image thereof (Line average) was acquired at a depth of 12 bit.

The above-described solution administration and all image acquisition procedures were conducted in a dark room maintained at a constant temperature (24°C) for 24 hours.

### (Experimental result)

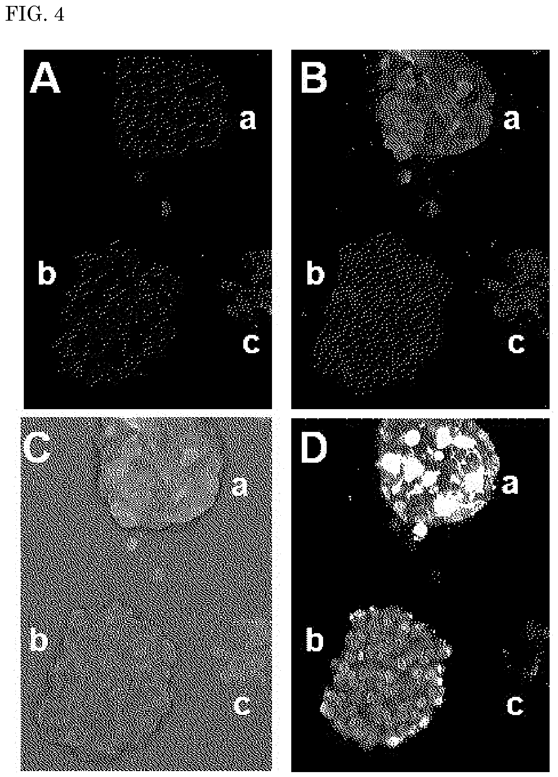

Fig. 4 shows the result of administration of a fluorescently labeled L-glucose derivative 2-NBDLG to insulin-producing tumor cells (MIN6) . 2-NBDLG is taken up into a cell cluster consisting of many three-dimensionally accumulated cells and exhibiting abnormal nuclei. A fluorescence image (excitation 488 nm, emission 500 to 580 nm) before administration of 2-NBDLG is shown in A. The presence of cells is confirmed by faint autofluorescence. B represents a fluorescence image obtained just after cessation of perfusion of cells with a HEPES solution containing 100 µM 2-NBDLG for 3 minutes and subsequent washout with a HEPES solution containing no 2-NBDLG. In the cell cluster (a) on the picture, intense uptake of 2-NBDLG is recognized. C shows overlay of a differential interference microscope (DIC) image and the fluorescence image B. For two cells scattered below the cell cluster (a), degraded condition is confirmed from the DIC image. D shows an image of nuclear staining using DAPI. Of the cell clusters (a) and (b) appearing to be very similar in the DIC image, cells in the cell cluster (b) manifest a normal nucleus morphology, while the cell cluster (a) contains cells emitting strong fluorescence and revealing an abnormal nucleus morphology. Remarkable uptake of 2-NBDLG is recognized only in the cluster (a). A to D are confocal microscopic images. These are described further in detail below.

Both the cell cluster (a) and the cell cluster (b) are an aggregate of a lot of mouse insulinoma cells (MIN6) cultured for 11 days. The perfusion solution applied to these cells was switched by an operation of a electromagnetic valve from the HEPES solution for image acquisition containing 2.8 mM glucose to a solution prepared similarly but allowing the same solution to contain 2-NBDLG (100 µM), which was then administered extracellularly for 3 minutes, and then the solution was returned to the HEPES solution for image acquisition so that the 2-NBDLG solution was washed away. When 2-NBDLG is taken up into cells, it is reflected to a difference in the fluorescence intensity before and after administration. The image A and the image B are green channel images (detection wavelength range: 500 to 580 nm) capturing green fluorescence emitted from 2-NBDLG. In image A (fluorescence image just before administration), cells emit a faint green fluorescence since they have autofluorescence originated from flavoproteins and the like. Its degree varies depending on the cell, and cell cluster (c) reveals slightly stronger autofluorescence, suggesting somewhat poor condition. In the image B (fluorescence image just after administration), cells remarkably taking up 2-NBDLG are recognized in the cell cluster (a). Since a fluorescent glucose derivative usually does not enter into a nucleus, nuclei of these cells are translucent. In contrast, as observed in the cell cluster (b), even a cell cluster consisting of exactly the same kind of tumor cells and similarly starting three-dimensional accumulation manifests only a slight uptake of 2-NBDLG. The cell cluster (c), of which autofluorescence is slightly stronger from before administration, shows almost no increase in the fluorescence intensity after administration. The image C shows superposition of the green channel image just after administration and the differential interference (DIC) image. It is understood that there is a significant difference in the state of uptake of 2-NBDLG between the cell cluster (a) and the cell cluster (b). The image D is a nuclear staining image obtained by administering 4,6-diamidino-2-phenylindole dihydrochloride (DAPI) to cells after cessation of the experiment. If the image B and the image D are compared, it is understood that 2-NBDLG uptake is scarcely observed in a cell cluster, in which no abnormal nuclei emerged in the central region and a nucleus morphology similar to that of a normal cell is seen (cell cluster (b)), and in a cell cluster at a stage of two-dimensionally accumulated planar conditions (cell cluster (c)). On the other hand, it is understood that cells strongly taking up 2-NBDLG appeared among cells in the cell cluster (a) so developed to a stage as to generate abnormal nuclei in the central region. Here, since the image D is taken under the condition where the focal depth of the confocal microscope in the image D is adjusted to a focal depth at which the most intense uptake of DAPI into abnormal nuclei is seen, the image D is a tomographic image at a section different from the focal depth of the images A to C. That is, according to the xyz-scanning, it has been suggested, in a tumor cell cluster, that a layer where abnormal nuclei emerge and a layer where a strong uptake of 2-NBDLG is seen are not identical. This becomes further clearer in Example 4 below. Though the cell cluster (c) in the images A, B and D is out of the focal depth range of the confocal microscope, the above descriptions on the cell cluster (c) are confirmed at a separate focal depth.

These results teach the following matters. Based on nuclear staining images visualized by DAPI which is administered for the purpose of knowing the degree of malignancy of tumor cells, the tumor cell cluster (a), which takes up 2-NBDLG, contains many cancer cells showing a nucleus morphology with a high degree of malignancy such as an extremely large nucleus, whereas the tumor cell cluster (b), which consists exactly of the same kind of tumor cells but scarcely takes up 2-NBDLG, contains cancer cells showing no such abnormal nucleus morphology (comparison of Fig. 4B and Fig. 4D). Therefore, if a properly designed fluorescently labeled L-glucose derivative is used, a difference in the condition of cells contained in cancer tissue consisting of the same kind of cancer cells can be visualized and evaluated according to a difference in the uptake of an L-glucose derivative.

### Example 4: Imaging of tumor cell cluster composed of mouse insulinoma cell (MIN6) using 2-NBDLG and 2-TRLG

### (Experimental methodology)

### (1) Preparation of mouse insulinoma cells (MIN6)

It was carried out in the same manner as in Example 3.

### (2) Preparation of 2-NBDLG solution and 2-NBDLG + 2-TRLG mixture

### Preparation of 2-NBDLG + 2-TRLG mixture

A 0.1 mg 2-TRLG vial was transferred into a desiccator and the temperature thereof was returned to room temperature. Since 2-TRLG is poorly soluble in water, the whole quantity of this 2-TRLG vial was dissolved using 65 µL of dimethyl sulfoxide (DMSO, Nacalai Tesque No. 13408-64) just after opening in a dark place. The 2-NBDLG solution was prepared in the same manner as described in Example 3 so as to give a final concentration of 100 µM just before an experiment, and the above-described 2-TRLG/DMSO solution was added to this by a method according to the non-patent document 16, to obtain a 2-TRLG concentration of 20 µM. Specifically, 6.5 mL of the above-described 2-NBDLG solution was prepared beforehand and stirred vigorously. Then, 40 µL of DMSO was added to a 2-TRLG vial container using a RAININ chip (Mettler Toledo) in which adhesion to the tip wall is ignorable, and stirred with the vial container by a mixer to cause dissolution thereof. 2-TRLG adhered to the wall was collected to the bottom by spinning down, the whole quantity thereof was removed by PIPETMAN using the same chip, and dissolved in the 2-NBDLG solution being stirred. 2-TRLG slightly remaining in the 2-TRLG vial container was recovered using 25 µL of DMSO in the same manner as described above, and further dissolved in the 2-NBDLG solution. By the above-described method, a mixed solution of 100 µM 2-NBDLG + 20 µM 2-TRLG (final concentration) was prepared with good reproducibility.

### (3) Method for administering 2-NBDLG + 2-TRLG mixture and image acquisition protocol

### (a) Administration of 100 µM 2-NBDLG + 20 µM 2-TRLG mixture

A 100 µM 2-NBDLG + 20 µM 2-TRLG mixture was administered for 3 minutes in a condition completely covering the whole tumor cell cluster. The state of the solution covering the whole cell cluster was confirmed each time without fail by fluorescence images in the green and red wavelength ranges during administration and acquisition of a transmitted light image. By stopping application of the fluorescent glucose mixture and switching simultaneously to the solution to a no fluorescent glucose-containing HEPES solution, the background fluorescence became ignorable level in 1 minute.

### (b) Details in image acquisition condition

By the method according to Example 3, image acquisition before administration of the mixture was started just after starting an experiment, and from then on, the fluorescence images in the blue, green and red wavelength ranges and a transmitted light image were acquired every 2 minutes until completion of the experiment. Regarding laser used, DAPI used for nuclear staining was excited by using 405 nm diode laser (AOTF 20%) and 2-NBDLG and 2-TRLG were excited by using 488 nm argon laser (output: 50 %, AOTF 28%). The scan speed was 400 Hz.

For fluorescence detection, three fluorescence detectors were used as described below. For detection of blue fluorescence by DAPI, a photomultiplier detector (PMT)1 was used in the wavelength detection range of 415 to 500 nm (detection sensitivity: 750 V), for detection of green fluorescence by 2-NBDLG, PMT2 was used in the wavelength detection range of 500 to 580 nm (detection sensitivity: 670 V), and for detection of red fluorescence, PMT3 was used in the wavelength detection range of 580 to 740 nm (detection sensitivity: 670 V). Here, most of the fluorescence of 2-TRLG is present in the wavelength longer than 580 nm and only a small fluorescent component is present in the wavelength less than 580 nm, thus, the fluorescence intensity in the range of 500 to 580 nm of PMT2 is barely influenced by 2-TRLG. Therefore, when the amount of detection of 2-TRLG in cells increases, its effect emerges mainly as an increase in the fluorescence intensity in the range of 580 to 740 nm of PMT3. On the other hand, although 2-NBDLG has the maximal fluorescence intensity around 540 to 550 nm, it has fluorescence in the range of 580 to 740 nm as well. Therefore, when the amount of detection of 2-NBDLG within the cell increases, the fluorescence intensity in the range of 580 to 740 nm detected by PMT3 also increases in addition to an increase in the fluorescence intensity in the range of 500 to 580 nm detected by PMT2. When only the detection amount of 2-NBDLG within the cell increases exclusively and 2-TRLG does not invade cells, the ratio of the fluorescence intensity of 2-NBDLG in the fluorescence wavelength range of 580 to 740 nm detected by PMT3 to the fluorescence intensity of 2-NBDLG in the fluorescence wavelength range of 500 to 580 nm detected by PMT2 shows a fixed relation depending on the concentration 2-NBDLG, the sensitivity profile of a detector, and the intensity of excitation light. In contrast, when 2-NBDLG is taken up into cells, if 2-TRLG also invades the cells in addition, an additional increase in the fluorescence intensity is detected compared with a predicted increase in the fluorescence intensity relative to before administration, which is derived from the above-described relation for the fluorescence intensity of 2-NBDLG in the fluorescence wavelength range of 580 to 740 nm detected with PMT3. The upper limit of the increase in the fluorescence intensity is informed from the ratio of the fluorescence intensity of the administered 100 µM 2-NBDLG + 20 µM 2-TRLG mixture in 500 to 580 nm range to the fluorescence intensity in 580 to 740 nm range, and the lower limit is the ratio of the fluorescence intensity in 500 to 580 nm range to the fluorescence intensity in 580 to 740 nm range when only 2-NBDLG is taken up into cells, and in reality, the increase in the fluorescence intensity varies between the lower limit and the upper limit depending on the extent of invasion of 2-TRLG into cells. In an actual protocol, the apparatus settings (namely, excitation light intensity and detector sensitivity) for acquisition of fluorescence in 580 to 740 nm range of PMT3 are adjusted so that an increase in the fluorescence intensity can barely be detected if 2-NBDLG is solely taken up into cells and 2-TRLG does not invade cells. In this way, when even a slight amount of 2-TRLG invades cells, the invasion can be detected immediately.

In selection of the above-described blue (415-478 nm), green (500 to 580 nm) and red (580 to 740 nm) fluorescence wavelength ranges for detection, a beam splitter at 500 nm (RSP500), which is capable of simultaneous detection of green fluorescence of 2-NBDLG and red fluorescence of 2-TRLG, is used. A beam splitter for 405 nm is fixed at 415 nm in the Leica SP5 system used. In fluorescence excitation, a sequential scanning mode (between frame scanning mode) was used in which a DAPI image by excitation at 405 nm was first acquired by a detector PMT1, then the image acquisition by 488 nm excitation follows, and in excitation at 488 nm, a green fluorescence image of 2-NBDLG and a red fluorescence image of 2-TRLG were acquired simultaneously by activating both the detectors PMT2 and PMT3 at the same time.

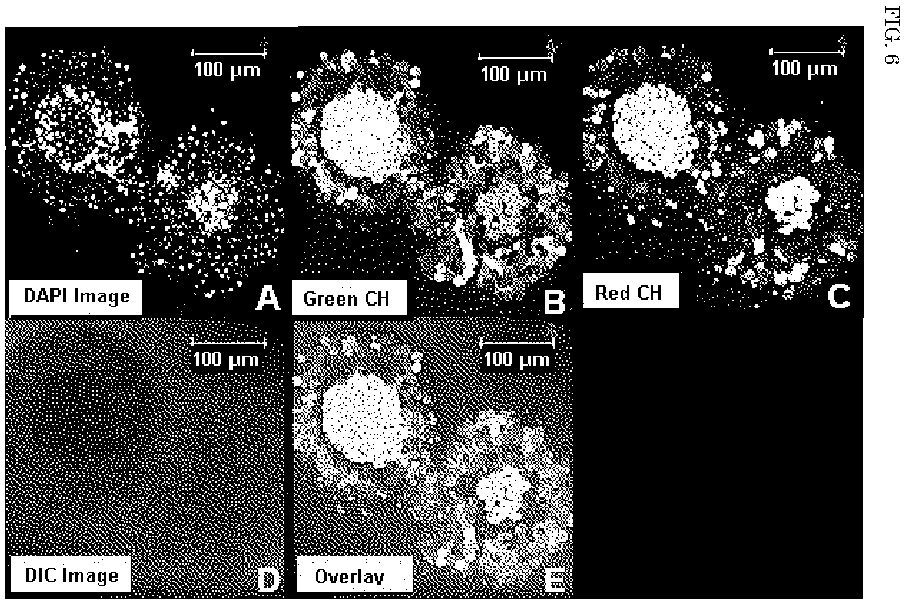

In actual image acquisition, for the purpose of time-lapse capturing of a change in the fluorescence of a cell at each depth in a three-dimensional cell cluster, xyzt scanning was performed, that is, xyz scanning was repeated every 2 minutes from 3 minutes before initiation of the administration of a fluorescently labeled glucose derivative until cessation of the administration protocol, in a way that a galvano stage of a confocal microscope was repeatedly moved in the z-axis direction with a constant distance for each above-described xy scanning. In this procedure, to know a difference in the response of cells depending on a difference in the depth in a cell cluster while minimizing fluorescence photo-bleaching by repeated irradiation with laser, the distance of movement of a stage along the z-axis direction was set to every 8 to 10 µm. Specifically, in the example in Fig. 6, starting from Z position corresponding to almost the right above the glass surface, scanning was performed one by one at a different depth displaced toward the z-axis direction by 8 to 10 µm sequentially, 2 or 3 pieces in total, and this operation was repeated every 2 minutes or 4 minutes. In this method, a 40x oil objective lens was used, and a perfusion chamber enabling rapid liquid exchange and sample exchange was used, and a cover slip on which cells were adhered was tightly contacted onto the glass surface above an inverted microscope stage and the sample was observed from the bottom. Therefore, due to a limitation of a high resolution objective lens with a short working distance touching to the glass, focusing at an upper structure of thick tumor cluster is limited toward the z-axis. The above-described distance of movement of a stage along the z-axis direction is determined in view of this point as well. The whole structure of a tumor cluster can be confirmed by replacing the lens to a low magnification lens before starting the administration protocol or in a separate experiment.

### (Experimental result)

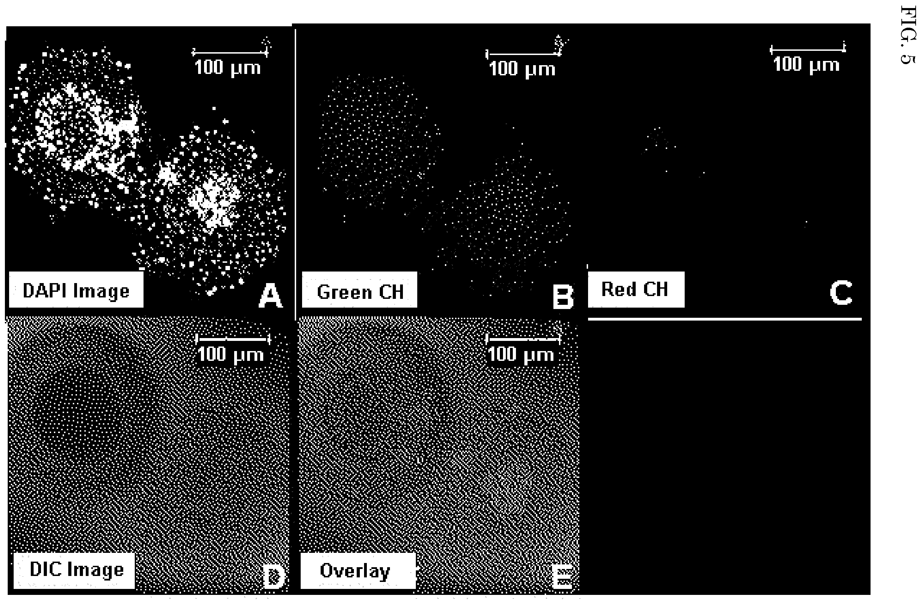

A 100 µM 2-NBDLG + 20 µM 2-TRLG mixture was administered to a developed insulin-producing tumor cell cluster (MIN6) for 3 minutes, and the cells were observed by a laser confocal microscope, and the results are shown in Figs. 5 to 9. (Fig. 5 to Fig. 7) Fig. 5 to Fig. 7 show fluorescence images and DIC images before administration of a 100 µM 2-NBDLG + 20 µM 2-TRLG mixture, at a time point 2 minutes after administration and at a time point 4 minutes after administration, respectively. In the image A (nuclear staining image with DAPI), the presence of cellular nuclei showing abnormal accumulation of DAPI at the central region is recognized in the left and right tumor clusters. The image B is a green channel image (500 to 580 nm) reflecting uptake of 2-NBDLG into cells. A fluorescence image taken by a confocal microscopy at one tomographic section within the tumor cluster is exhibited. These are described in detail below.

Fig. 5 is a confocal microscope tomographic image of a cell cluster composed of many insulin-producing tumor cells (MIN6) before administration. A is a nuclear staining image with DAPI. Very intense nuclear staining images are observed around the central region of the upper left cell aggregation and the lower right cell aggregation. B is a fluorescence image in green channel (500 to 580 nm) before administration. Autofluorescence is recognized. C is a fluorescence image in red channel (580 to 740 nm). In this wavelength range, autofluorescence is usually extremely small. D is a differential interference (DIC) image. E is a superimposed image of A-D.

Fig. 6 is an image obtained 2 minutes after cessation of administration of 100µM 2-NBDLG + 20µM 2-TRLG solution. A is a nuclear staining image with DAPI. B is a fluorescence image in green channel reflecting uptake of 2-NBDLG. Intense uptake is recognized at the central region of the cell cluster, and in the outer surrounding regions, cells manifesting various extents of uptake are scattered. In regions directly surrounding the central region, cells manifesting scarce uptake are also observed. C is a fluorescence image in red channel, which strongly reflecting uptake of 2-TRLG (the proportion of contribution of 2-TRLG and 2-NBDLG to the fluorescence intensity detected in a red channel is understood in the same manner as for the proportion of contribution of 2-TRLG and 2-NBDG to a red channel). Excepting a strong uptake at the central region, a bipolarized tendency to either intense staining or very weak staining is found in the surrounding regions. D is a DIC image. In the left cell cluster, the central region is dented in the form of a doughnut. E is an overlaid image. It is found that there are cells not taking up a fluorescent L-glucose derivative, green cells taking up only 2-NBDLG, yellow cells taking up 2-NBDLG and 2-TRLG, and red cells in which fluorescence by 2-TRLG remains.

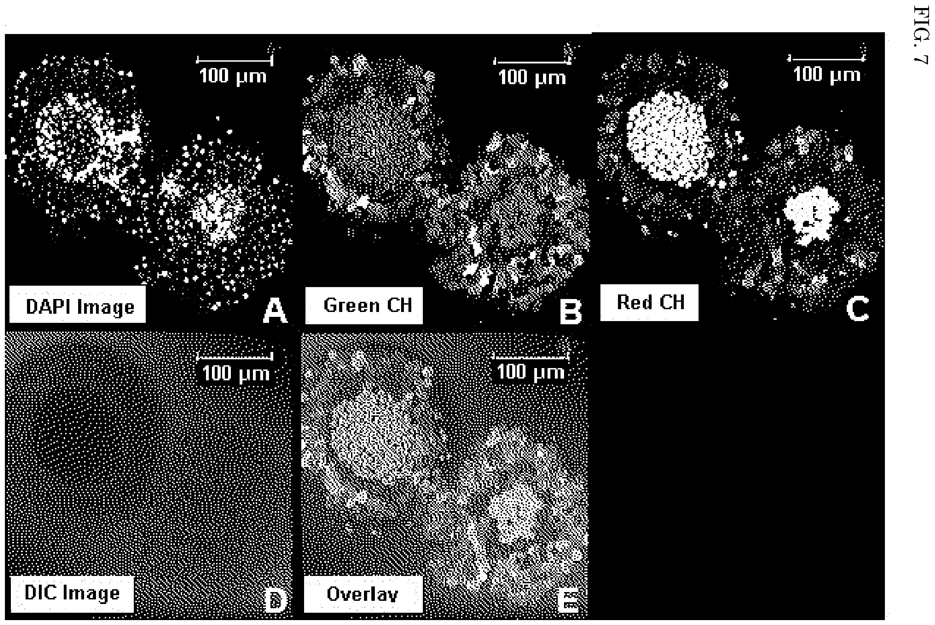

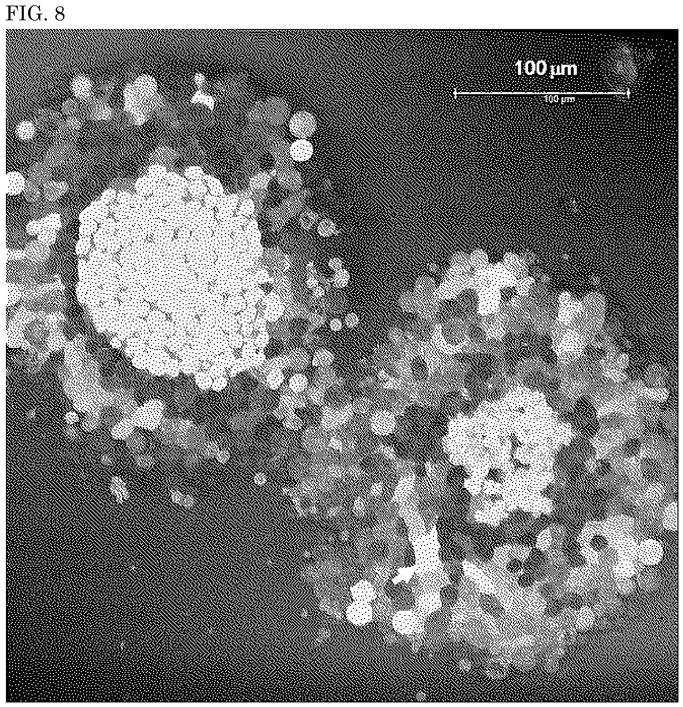

Fig. 7 is an image obtained 4 minutes after cessation of administration of a 100µM 2-NBDLG + 20µM 2-TRLG solution. At the central region of the cell cluster exhibiting an abnormal nucleus morphology, the fluorescence intensity is weaken to a considerable extent since 2-NBDLG is already eliminated (B), but the fluorescence by 2-TRLG once taken up is not weakened yet (C). As a result, the color tone of the central region is different from that in Fig. 6. Cells exhibiting a strong blue color by DAPI (A) overlap with green cells showing good uptake of 2-NBDLG in the central region of the cell cluster, but not necessarily overlap with in the surrounding regions. Details of the changes in the green channel and the red channel are described referring to Fig. 8 to Fig. 9.

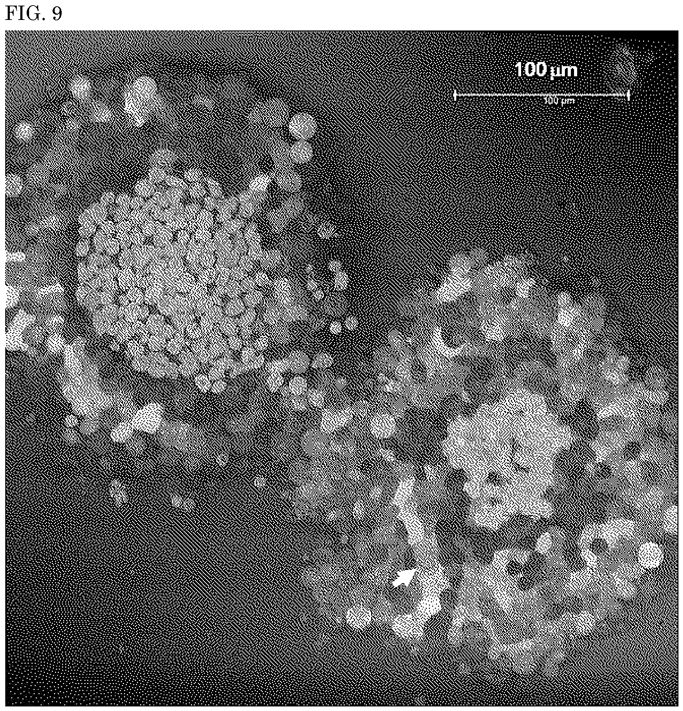

As described above, it has been found by using 2-NBDLG that it is possible to visualize and discriminate tumor cells showing various 2-NBDLG uptake conditions such as cells barely taking up 2-NBDLG as seen in cells located in the regions just outside of the central region of the cell cluster, and cells showing very well uptake and moderate uptake of 2-NBDLG in the regions outside thereof, even in the same cell cluster. A cell indicated by a white arrow in Fig. 7 cannot be discriminated from surrounding faintly glowing cells in green color at a comparable level solely by uptake of 2-NBDLG in this figure, however, when observing the state of uptake of 2-TRLG administered simultaneously (see, Figs. 8 and 9), its nature quite distinct from the surrounding cells is discernible. The image C is a red channel image (580 to 740 nm) mainly reflecting 2-TRLG uptake into cells. The image D is a DIC (differential interference contrast) image of a three-dimensionally highly accumulated tumor cluster. In the left tumor cell cluster, the cell cluster is raised and its central region is somewhat dented in the form of a crater. In the right cell cluster, its height is somewhat smaller as compared with the left cell cluster. Unlike the fluorescence image using a pin hole, cells present on different focusing planes can be observed in the DIC image though somewhat defocused. The image E is an A-D superimposed image.

### (Fig. 8 and Fig. 9)

Fig. 8 is an image obtained by extracting the fluorescence images in green and red channels at a time point 2 minutes after cessation of administration of 100 µM 2-NBDLG + 20 µM 2-TRLG mixture and superimposing the fluorescence images and the DIC image. The central region of the cell cluster takes up both 2-NBDLG and 2-TRLG well, thereby showing yellow color. On the regions just outside thereof, dark cells taking up neither of them are present. Cells taking up 2-NBDLG to various extents surround further outside thereof. The cells showing only red color are cells which take up 2-NBDLG and 2-TRLG and rapidly eliminated the former by washout. Cells showing different focal depths around the cell cluster are not imaged at the focal depth of this figure owing to the confocal effect in the fluorescence image. Thus, in addition to visualization of the presence of cells taking up green fluorescence-emitting 2-NBDLG to various extents as seen in Fig. 6, cells taking up 2-TRLG as well, thereby showing yellow color, are recognized. Almost all of the cells showing yellow color changed their fluorescent color tone to a color in the red color family, as is understood by the view at a time point 4 minutes after cessation of administration shown in Fig. 9.

For its explanation, there is a hypothesis as described below, based on another experiment using single neuronal cells. When 2-TRLG is taken up in combination with 2-NBDLG into neuronal cells of which cell condition is known to be not completely dead but degraded moderately, the time course for the uptake of 2-TRLG is slower than the time course for the uptake of 2-NBDLG, that is, 2-TRLG enters slowly, and instead, once entered into a cell, 2-TRLG needs a longer time than 2-NBDLG, from initiation of washout until elimination out of the cell, even if the amount of 2-TRLG is smaller than that of 2-NBDLG. Therefore, the tumor cells taking up both 2-TRLG and 2-NBDLG into cells to show yellow color eliminate green 2-NBDLG firstly after initiation of washout, leaving red 2-TRLG within cells. Actually, cells taking up red 2-TRLG to various extent, which are recognized in Fig. 8, mostly look yellow if the colors of these cells are checked during administration of a 2-NBDLG + 2-TRLG mixture or before cessation of two average scanning in the first image acquisition after cessation of administration.

Cells indicated by a white arrow in Fig. 8 still show strong yellow color at a time point 2 minutes after cessation of administration, and it is understood that red 2-TRLG is taken up in addition to 2-NBDLG differing from the surrounding cells, however, as is apparent from the color of cells likewise indicated by a white arrow in Fig. 9 at a time point 4 minutes after cessation of administration, green 2-NBDLG flows out of the cell rapidly during this 2 minutes and 2-TRLG remains in a large amount within the cell, resulting in red color. That is, 2-TRLG is capable of playing a role of informing the fact that a large amount of 2-NBDLG was taken up once in the past like a memory for some time. If 2-TRLG is not used in combination with 2-NBDLG, it is impossible to know that the cell indicated by a white arrow in Fig. 7 is a cell showing an uptake pattern which is distinct from that of the surrounding cells showing pale green color. This cell is recognized to be in a cell condition distinct from that of the surrounding cells at the instance when checking the uptake state of simultaneously administered 2-TRLG in combination.

When investigating the uptake of a fluorescently labeled glucose bringing into contact with tumor cells under variety of actual conditions other than those in a laboratory, it seems conceivable that investigating the uptake state along with a time course strictly is difficult, thus, to use red 2-TRLG and green 2-NBDLG simultaneously is advantageous in discriminating the cell condition of tumor cells showing heterogeneity.

Fig. 9 was made by superimposing the fluorescence images in green and red channels at a time point 4 minutes after cessation of administration of 100 µM 2-NBDLG + 20 µM 2-TRLG mixture on the DIC image. Cells at the central region of the cell cluster and a cell indicated by a white arrow show yellow color by green 2-NBDLG and red 2-TRLG at a time point in Fig. 8, however, at a time point in this figure after passage of 2 minutes, green 2-NBDLG is already eliminated out of the cell, and red color is stronger than that in Fig. 8 due to relatively delayed elimination of red 2-TRLG. As observed at the outer edge of the cell cluster, in some cells that are already in red in Fig. 8, even red 2-TRLG is eliminated in this figure. The cell group at the central region of the tumor cell cluster shows a color in the red color family in Fig. 9 (pink in Fig. 7 by mixing with blue color of DAPI), but since this cell group showed yellow color in Fig. 8, therefore, it is understood that these cells are in a cell condition in which both red 2-TRLG and green 2-NBDLG are taken up, and then green 2-NBDLG is eliminated rapidly within 2 minutes . On the other hand, among cells showing already red color in Fig. 8, for example, in some of spherical cells present on the outermost periphery of the cell cluster, it seems conceivable that 2-NBDLG flows out of the cell at a speed as fast as within 2 minutes after cessation of administration, and indeed the red color of many of these cells already disappears in Fig. 9. This means that 2-TRLG is eliminated out of the cell within 4 minutes in these cases. This strongly suggests that the membrane permeability of these cells is increased extremely, and it seems conceivable that these cells are in terminal state close to death in their cell condition.

When compared with such cells, it seems conceivable that the cell group showing a color in red color family at the central region of the tumor cell cluster in Fig. 9 might not be in fatal condition yet, and is not completely dead. This estimation is also supported by referring to the nuclear staining condition of these cells.

On the other hand, in Figs. 8 and 9, it is clearly discerned that dark cells taking up almost no fluorescently labeled glucose derivatives having L-glucose scaffold, that is, both red 2-TRLG and green 2-NBDLG, are present so as to surround the regions just outside of the central region of the cell cluster (regions looked black in Fig. 6B and Fig. 7B in which differential interference contrast (DIC) image is not overlaid) . As is understood from the DIC image, a possibility that cells are not present in this region is excluded. That is, a stereoselectivity of plasma membrane for glucose uptake in these cells is preserved as in normal cells. Possibility of visualization of the presence of tumor cells maintaining the condition of the plasma membrane corresponding to that of normal cells is expected to be useful in investigating the effectiveness of anticancer drugs and treatment. As described above, in the present invention, a fluorescently labeled glucose derivative having L-glucose scaffold is administered to a cell group including a developed tumor cluster, and a difference of the cell condition of individual tumor cells can be positively evaluated according to the extent of uptake thereof into individual cells, and additionally, the present invention is expected to be widely applicable, as a marker, to the evaluation of effectiveness of a drug and radiation therapy, in a way of making such cells distinct by their taking up no such fluorescently labeled glucose derivative having L-glucose scaffold into the cells.

### Example 5:

A difference in the uptake of 2-NBDLG (200 µM) and 2-NBDG (200 µM) into mouse insulinoma cells (MIN6) each applied for 5 minutes was analyzed quantitatively by a fluorescence microplate reader Flex Station (manufactured by Molecular Devices Corporation) in the condition whether or not various inhibitors are present.

### (1) Preparation of cells

The well used for measurement was a 96-well clear bottomed well plate µ CLEAR-PLATE (Greiner bio-one, BLACK, 96 well, #655090) having longitudinally A to H eight lines and laterally 1 to 12 twelve columns, and MIN6 cell suspension prepared at a proportion of 6 x 10⁵ cells/mL was seeded uniformly into wells from line B to line G at the third column and the fifth column each in an amount of 15µL. For medium exchange, the half quantity was exchanged every two days in 0 to 4 DIV (days in vitro), and from 5 DIV or later, exchanged every day, and on days 10 to 13 (10 to 13 DIV), the cells were subjected to an experiment. Line A and line H were used as a control for checking correct washout, without seeding cells. In the fourth column, cells were not present, and wells in this column were used as a blank containing only Krebs Ringer buffer solution (KRB, containing 0.1 mM gap junction inhibitor carbenoxolone and 5.6 mM glucose).

### (2)Measurement of uptake

Evaluation was done in a way that 2-NBDLG or 2-NBDG was charged in each tube of a 8-strip PCR tube, and sucked using a 8-channel pipette and dropped simultaneously into wells. In this way, administration to wells for 2-NBDLG and to wells for 2-NBDG can be done simultaneously, making reliable evaluation possible. As described above, cells are present in 12 wells in the third and the fifth two columns, and these were divide into 4 groups each having 3 wells according to a difference either 2-NBDLG or 2-NBDG and whether an inhibitor is present or absent. Specifically, when checking the effect by the presence or absence of a GLUT inhibitor Cytochalasin B (10 µM) on the uptake of 2-NBDLG (200 µM) and 2-NBDG (200 µM), first, in wells, which are planned to be measured in the presence of Cytochalasin B, Cytochalasin B was added 5 minutes prior to the administration of the fluorescently labeled glucose derivative. Before administration of a fluorescently labeled glucose derivative, the autofluorescence was measured in each well. The measurement was done with Flex Station in Well Scan Mode and in the Bottom Read position at Ex 470 nm, Em 540 nm, Cut off 495 nm, Averaging 3, and Photomultiplier sensitivity: high. In Well Scan Mode of the fluorescence microplate reader Flex Station, one well was divided into nine observation areas and these are measured independently. The presence of approximately 5000 cells in each of 9-divded areas was separately confirmed by Z section measurement of a real-time deconvolution microscope.

2-NBDG and 2-NBDLG were administered with or without Cytochalasin B (37°C, 5 minutes), and after cessation of administration, an operation of adding 250 µL of a KRB solution containing no fluorescently labeled glucose into 50 µL of a solution in a well was repeated 7 times while measuring with a stopwatch, thereby performing washout until the fluorescence intensities of wells in line A and line H set as a control group became equal to the fluorescence intensity of the blank well containing no cell. This process required 7 minutes, and even if cells having broken plasma membrane condition are brought into contact with 2-NBDG and 2-NBDLG, and even if these compounds are once taken up into the cell, these compounds are already eliminated out of the cell and are washed away in measurement, therefore, contribution to an increase in the fluorescence intensity in the whole observation area was judged to be in an ignorable level. This was supported by a separate pharmacological inhibition experiment in which the increase in the fluorescence intensity nearly disappears in the presence of an inhibitor. The above-described method was carried out similarly in the case of other inhibitors. It was confirmed that the osmotic pressure of the Na⁺ -free KRB medium is unchanged by substituting choline for Na⁺.

### (Experimental result)

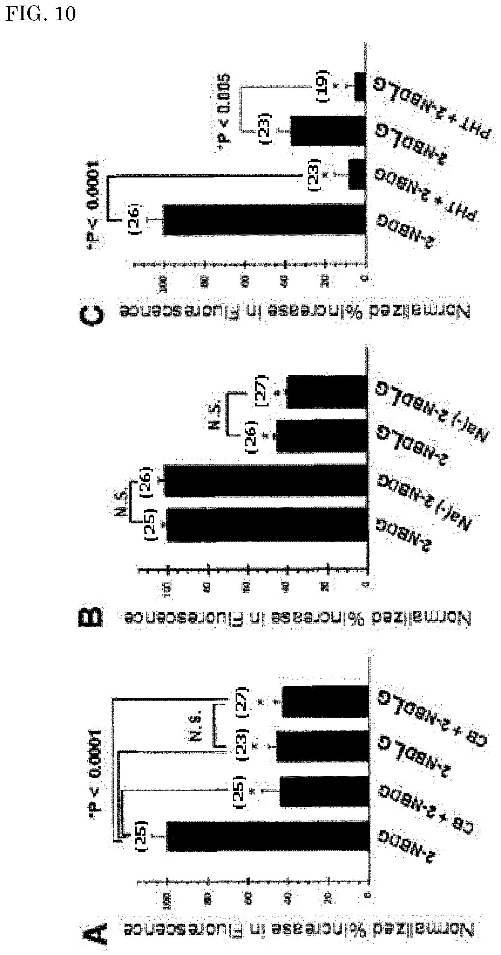

Fig. 10 shows the effects of inhibitors on the uptake of 2-NBDG and 2-NBDLG into mouse insulinoma (MIN6) cells quantitatively evaluated by a fluorescence plate reader.
(A) Uptake of 2-NBDG showed a significant decrease by adding a GLUT inhibitor Cytochalasin B (10 mM, CB), showing uptake via a glucose transporter GLUT. In contrast, uptake of 2-NBDLG did not show a significant decrease (N.S.) by Cytochalasin B, suggesting an uptake not via GLUT. An asterisk represents a significant low value with respect to an increase in the fluorescence intensity by administration of 2-NBDG (p< 0.0001, ANOVA, Bonferroni/Dunn).
(B) Whether 2-NBDG and 2-NBDLG are taken up via SGLT, a glucose transporter co-transporting a Na⁺ ion and glucose, or not was investigated. Both 2-NBDG and 2-NBDLG showed no significant difference in the uptake into a cell between a case that fluorescent glucose dissolved in a buffer containing a Na⁺ ion was administered and a case that fluorescent glucose dissolved in a buffer containing no Na⁺ ion (Na(-)) was administered. Since SGLT does not function in the absence of a Na⁺ ion, it is suggested that SGLT barely contributes to the uptake of NBDLG into MIN6 cells.
(C) Both 2-NBDG and 2-NBDLG underwent inhibition of the uptake into a MIN6 cell by Phloretin (150 pM, PHT), a common inhibitor for both GLUTs and water channels. It is suggested that the uptake of 2-NBDLG into a cell is mediated by a selective pathway inhibited by PHT.

In a separate experiment, inhibition of 2-TRLG uptake into a cell by PHT was tested, but there was no inhibition.

### Example 6: Imaging by administration of 2-NBDLM + 2-TRLG mixture to acutely dissociated neurons

### (Experimental methodology)

### (A) Experiment was done by using materials and solutions having the following compositions.

### (Composition of HEPES solution for confocal image acquisition)

NaCl 150 mM, KCl 5 mM, MgCl₂ 1 mM, CaCl₂ 2 mM, HEPES 10 mM, pH was adjusted to 7.4 by 1M Tris (2-amino-2-hydroxymethyl-1,3-propanediol) solution. The glucose concentration was 10 mM. For the purpose of inhibiting entrance and elimination of fluorescently labeled mannose via a gap junction/hemichannel, 0.1 mM Carbenoxolone (SIGMA #C4790) was added.

### (Composition of Ringer's solution for dissociation)

NaCl 124 mM, NaHCO₃ 26 mM, KCl 5 mM, KH₂PO₄ 1.24 mM, CaCl₂ 2.4 mM, MgSO₄·7H₂O 1.3 mM, Glucose 10 mM, adjusted to pH7.4 by 95%O₂-5%CO₂ aeration.

### (Poly-L-lysine coating of cover slip (for neuronal cell))

A solution prepared by dissolving 5 mg of poly-L-lysine hydrobromide (SIGMA P6282) in 50 mL of 0.15 M boric acid (adjusted to pH 8.4 with NaOH) was stocked and refrigerated. This stock solution was diluted at 1/500 to 1/1000 with 0.15 M boric acid, and a cover slip (13 mm x 22 mm, Matsunami No. 0 glass) was immersed in the diluted solution and allowed to fix at room temperature for 20 minutes, then, rinsed with distilled water and naturally dried.

### (B) Preparation of 2-NBDLM solution and 2-NBDLM + 2-TRLG mixture

Since 2-NBDLM is a stereoisomer of 2-NBDLG and both compounds have the same molecular weight, these compounds were prepared by basically the same method as for preparation of 2-NBDLG liquid and 2-NBDLG + 2-TRLG mixture described in the prior publication WO2010/016587 of the present inventors. The concentration of a solution for the administration was as follows. The whole quantity of a 0.5 mg vial was dissolved in 14.6 mL of a HEPES solution for image acquisition to give a 2-NBDLM solution having a final concentration of 100 µM. The dissolving method was carried out similarly to that for preparation of a 2-NBDLG solution in Example 3. Since 2-TRLG is poorly soluble in water, 1 mg of 2-TRLG was first dissolved in a dimethyl sulfoxide (DMSO) solution to give a 2 mM 2-TRLG solution, then, a 100 µM 2-NBDLM + 20 µM 2-TRLG mixture was obtained by diluting this 2 mM solution 100-fold with a HEPES solution for image acquisition having the following composition prepared beforehand so as to contain 100 µM 2-NBDG.

### (C) Preparation of acutely dissociated neuronal cell

Acutely dissociated neuronal cells were prepared as described below according to the same method as described in the prior publication WO2010/016587 of the present inventors.

### (1) Preparation of mouse brain slice

Postnatal 13 to 17-day old (juvenile stage) C57BL/6J mice were anesthetized deeply with urethane (i.p. 1.6 g/kg), decapitated according to an ordinary method, then, brain was taken out while pouring a cold Ringer's solution (0°C) for dissociation, and placed on an ice-cold petri dish and immediately trimmed, then, coronally sectioned brain slices having a thickness of 400 or 500 µm were prepared by a linear slicer (Dosaka Pro7) in a cold Ringer's solution (0°C) . Then, the slice was allowed to restore for 1 hour at room temperature in a chamber (Acrylic incubation chamber manufactured by Harvard Apparatus) containing the above-described Ringer's solution circulating therein through which 95%O₂ -5%CO₂ had been aerated.

### (2) Enzymatic treatment of brain slices

The brain slice was immersed in an enzyme solution (31°C; kept at pH7.4 by aeration with 95%O₂-5%CO₂) prepared by dissolving a proteolytic enzyme Pronase (10mg/60mL) in the above-described Ringer's solution and enzymatically treated. After predetermined period, the enzymatic reaction was stopped by immediately immersing the brain slice in 50-100 mL of a 10 mM glucose-containing HEPES solution at room temperature.

### (3) Separation of mesencephalic substantia nigra pars reticulata

A 60 or 100 mm plastic dish having a silicon plate attached to the bottom was filled with a 10mM glucose-containing HEPES solution(room temperature, obtained by removing Carbenoxolone from a HEPES solution for confocal image acquisition), and the above-described brain slice was immersed in the solution. Two 27 gauge syringe needles were used to immobilize the brain slice, and left and right mesencephalic substantia nigra pars reticulata were punched out under a microscope with a 18 gauge syringe needle having a tip deformed into an ellipsoidal shape, and preserved at room temperature in the above-described HEPES solution (35mm culture dish).

### (4) Dissociation of neurons of substantia nigra pars reticulata

Neurons were dissociated by trituration with glass pipettes adjusted to have various point diameters, and allowed to adhere to a glass cover slip coated with poly-L-lysine.

### (D) Administration method of 2-NBDLM+2-TRLG mixture and image acquisition protocol in case of using real time confocal laser scanning microscope

Administration of a mixture was carried out in the same manner as the method of administration to MIN6 cells described in Example 3 (4) to (6).

### (Experimental result)

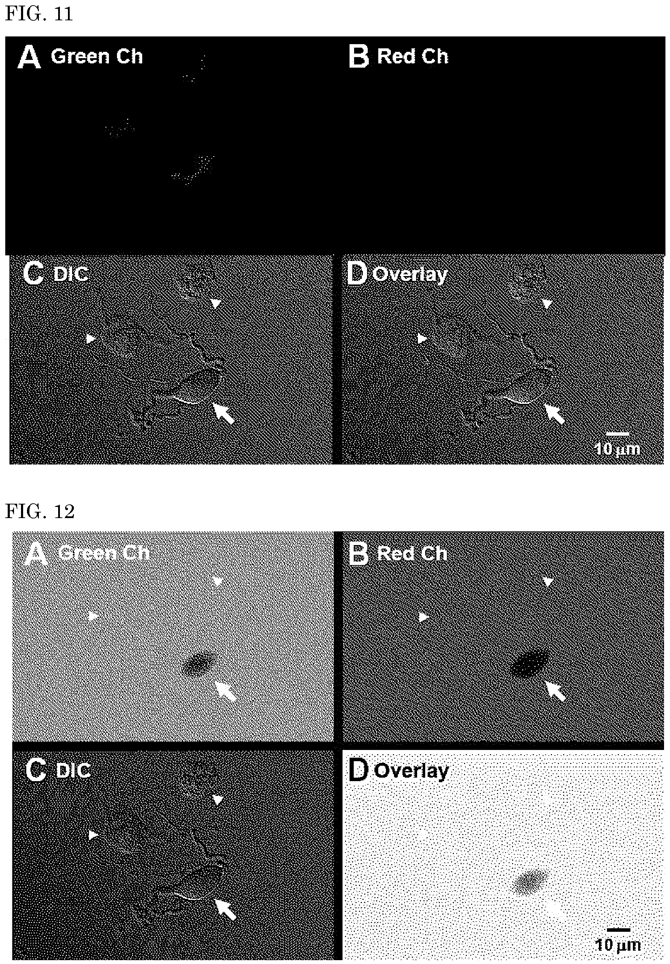

Figs. 11 to 15 show results of the administration of 100 µM 2-NBDLM + 20 µM 2-TRLG mixture to acutely dissociated neurons for 5 minutes observed by a laser confocal microscope.

### (Fig. 11 to Fig. 15)

Each represents before administration of a 100 µM 2-NBDLM + 20 µM 2-TRLG mixture, during administration thereof, at a time point 2 minutes after cessation of administration, at a time point 10 minutes after administration, and at a time point 22 minutes after administration, wherein (A) is a fluorescence image in green channel (500 to 580 nm), (B) is a fluorescence image in red channel (580 to 740 nm), (C) is a differential interference (DIC) image, and (D) is an overlayed image of ABC.

Fig. 11 shows autofluorescence images in green (A) and red (B) channel just before administering 2-NBDLM (100 µM) + 2-TRLG (20 µM) to neurons acutely dissociated from mouse mesencephalic substantia nigra pars reticulata, a differential interference(DIC) image (C), and an overlaid image thereof (Overlay, D).

Fig. 12 shows fluorescence images in green channel and red channel during administration of 2-NBDLM (100 µM) + 2-TRLG (20 µM), a differential interference image and an overlaid image (all are original images without processing). Since a healthy cell indicated by an arrow is not invaded by 2-TRLG during administration, a cell body region is expressed as a shadow in the fluorescence image in red channel (B) strongly reflecting the fluorescence of 2-TRLG. In contrast, when observing the fluorescence image in green channel (A) reflecting uptake of 2-NBDLM, it is found that the shadow of a cell body is smaller than that in red channel since 2-NBDLM is taken up into a cell during administration. In the overlaid image D, a cell indicated by an arrow shows green color in the cytoplasmic part excluding nucleus in the central area reflecting the fluorescence of 2-NBDLM. On the other hand, in two degraded cells indicted by arrow heads, a shadow of a cell body is not recognized during administration in any of green channel and red channel, and both 2-NBDLM and 2-TRLG completely invading a cell during administration is shown.

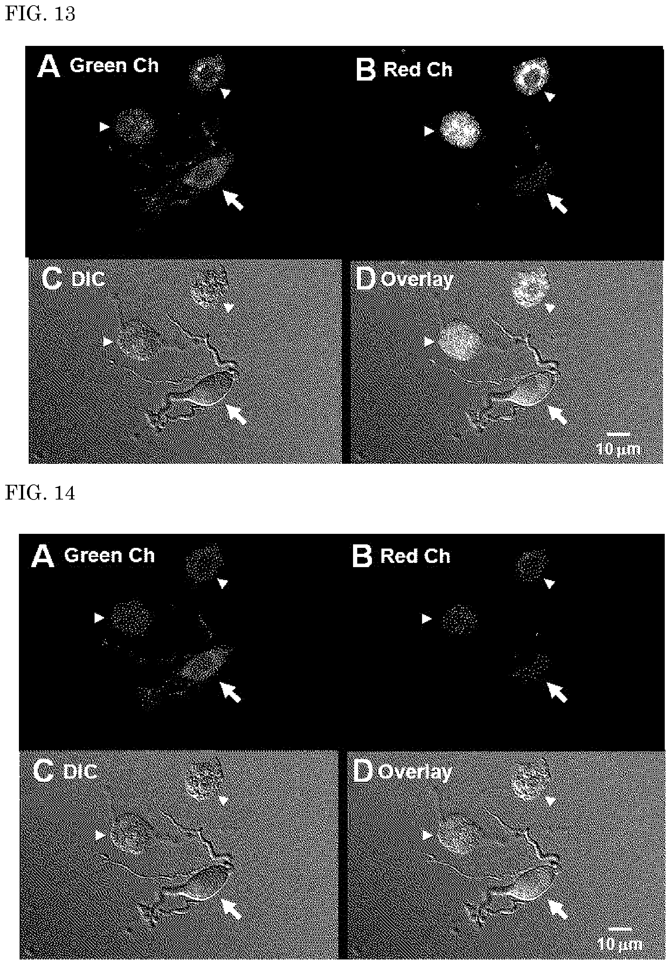

Fig. 13 shows images at a time point 2 minutes after cessation of administration of 2-NBDLM (100 µM) + 2-TRLG (20 µM) (fluorescence change is so expressed as to give facilitated visualization by weighting green channel and red channel at the same level) . When the green channel of A is viewed, it is found that all cells take up 2-NBDLM. However, when the red channel of B is viewed, 2-TRLG is not taken up into a healthy cell indicated by an arrow and it is shown that the plasma membrane is healthy, whereas two cells indicated by arrow heads show uptake of a large amount of 2-TRLG and it is understood that the plasma membrane is broken. As a result, in the overlaid image D, a cell indicated by an arrow shows green fluorescence in the cell body, whereas cells indicated by arrow heads show orange color by mixing of green color and red color. The fluorescence intensity in red channel of a cell indicated by an arrow seems slightly more intense as compared with before administration because an increase in fluorescent components in red channel of 2-NBDLM is visualized by image enhancement (details are described in [0096]).

Fig. 14 shows an image at a time point 10 minutes after cessation of administration of 2-NBDLM (100 µM) + 2-TRLG (20 pM). When the green channel A and the overlaid image D are observed, it is understood, in the case of a healthy cell indicated by an arrow, that 2-NBDLM represented by green fluorescence is preserved in the cell even 10 minutes after cessation of administration. In contrast, in two cells represented by arrow heads, it is found that the fluorescence in green channel and red channel observed intracellularly in the image at a time point 2 minutes after administration have already decreased significantly, and that 2-NBDLM and 2-TRLG once invaded the cell due to plasma membrane breakdown flowed out of the cell along with the washout elapsed time.

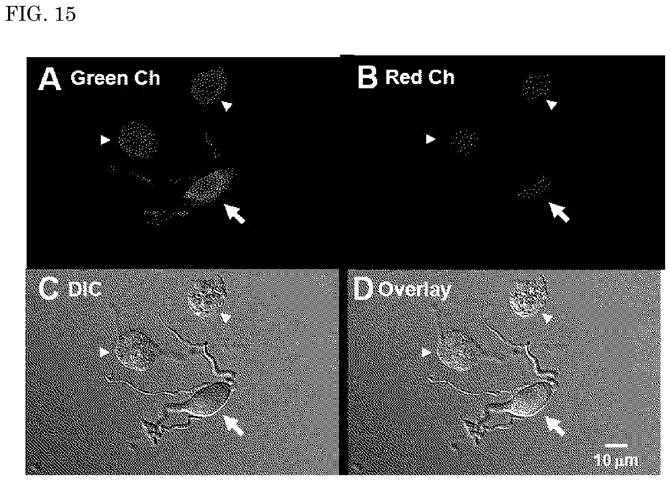

Fig. 15 shows an image at a time point 22 minutes after cessation of administration of 2-NBDLM (100 µM) + 2-TRLG (20 µM) . As apparent from A and D, the green fluorescence by 2-NBDLM is preserved in the cell even at a time point 22 minutes after cessation of administration. Figs. 13, 14 and 15 are all shown under exactly the same image processing conditions.

The above-described results denote that 2-NBDLM is taken up into normal mouse neurons.

### INDUSTRIAL APPLICABILITY

The present invention has industrial applicability since it can provide a method for accurately determining whether there are tumor cells or not.

## Claims

1. A method of detecting cancer cells *in vitro,* comprising the following steps:
(a) a step of bringing a composition containing an L-glucose or an L-glucose in which a hydroxyl group is substituted by an amino group or a fluorine atom, said L-glucose being linked to a 7-nitrobenz-2-oxa-1,3-diazole group or a 7-(N,N-dimethylaminosulfonyl)benz-2-oxa-1,3-diazole group via an amine or via a spacer represented by the formula -NH-(CH₂)ₙ-NH-, in which formula n is 2 to 20, or a salt form thereof into contact with a target cell *in vitro,*
(b) a step of detecting the L-glucose linked to the 7-nitrobenz-2-oxa-1,3-diazole group or 7-(N,N-dimethylaminosulfonyl)benz-2-oxa-1,3-diazole group present in the target cell by detecting fluorescence, and
(c) a step of detecting a cancer cell based on an increase in the fluorescence intensity in the target cell.

2. The detection method according to claim 1, wherein the L-glucose linked to a 7-nitrobenz-2-oxa-1,3-diazole group or a 7-(N,N-dimethylaminosulfonyl)benz-2-oxa-1,3-diazole group is obtained by linking a 7-nitrobenz-2-oxa-1,3-diazole group or a 7-(N,N-dimethylaminosulfonyl)benz-2-oxa-1,3-diazole group to the 2-position, 4-position or 6-position of the L-glucose.

3. The detection method according to claim 1 or 2, wherein the L-glucose linked to a 7-nitrobenz-2-oxa-1,3-diazole group or a 7-(N,N-dimethylaminosulfonyl)benz-2-oxa-1,3-diazole group is 2-[N-(7-nitrobenz-2-oxa-1,3-diazole-4-yl)amino]-2-deoxy-L-glucose or 2-deoxy-2-[N-7-(N',N'-dimethylaminosulfonyl)benz-2-oxa-1,3-diazole-4-yl)amino]-L-glucose.

4. The detection method according to any one of claims 1 to 3, wherein detection in the step (b) is conducted by imaging the target cell.

5. The detection method according to any one of claims 1 to 4, wherein the composition in the step (a) further contains another L-glucose or another L-glucose in which a hydroxyl group is substituted by an amino group or a fluorine atom, said another L-glucose being linked to sulforhodamine, wherein the L-glucose of the composition of step (a) and the said another L-glucose are differently taken up into cells and detected at different wavelengths, thereby excluding noises by the uptake attributable to cell membrane damages due to inflammation or physical injury, and by non-specific uptake of macrophage.

6. The detection method according to claim 5, wherein the composition contains 2-[N-(7-nitrobenz-2-oxa-1,3-diazole-4-yl)amino]-2-deoxy-L-glucose and 2-amino-2-deoxy-L-glucose linked to sulforhodamine 101 or sulforhodamine B at the 2-position by way of a sulfonamide bond.

7. The detection method according to claim 5 or claim 6, wherein detection in the step (b) is conducted by using a temporal change in the fluorescent color tone of the imaged cell as an index.

8. A compound for use in an *in vivo* diagnosing method for detecting cancer cells, wherein said compound is an L-glucose or an L-glucose in which a hydroxyl group is substituted by an amino group or a fluorine atom, said L-glucose being linked to a 7-nitrobenz-2-oxa-1,3-diazole group or a 7-(N,N-dimethylaminosulfonyl)benz-2-oxa-1,3-diazole group via an amine or via a spacer represented by the formula -NH-(CH₂)ₙ-NH-, in which formula n is 2 to 20, or a salt form thereof, wherein the diagnosing method comprises the steps of:
(1) administering said compound to a living body locally or systemically,
(2) performing imaging, and
(3) evaluating the uptake of said compound into a cell by detecting an increase in the fluorescence intensity, and thus detecting cancer cells.

9. The compound for use according to claim 8, wherein the L-glucose linked to the 7-nitrobenz-2-oxa-1,3-diazole group or 7-(N,N-dimethylaminosulfonyl)benz-2-oxa-1,3-diazole group is obtained by linking a 7-nitrobenz-2-oxa-1,3-diazole group or a 7-(N,N-dimethylaminosulfonyl)benz-2-oxa-1,3-diazole group to the 2-position, 4-position or 6-position of the L-glucose.

10. The compound for use according to claim 8 or 9, wherein the L-glucose linked to the 7-nitrobenz-2-oxa-1,3-diazole group or the 7-(N,N-dimethylaminosulfonyl)benz-2-oxa-1,3-diazole group is 2-[N-(7-nitrobenz-2-oxa-1,3-diazole-4-yl)amino]-2-deoxy-L-glucose or 2-deoxy-2-[N-7-(N´,N´-dimethylaminosulfonyl)benz-2-oxa-1,3-diazole-4-yl)amino]-L-glucose.

11. The compound for use according to any one of claims 8 to 10, wherein the imaging agent further contains another L-glucose or another L-glucose in which a hydroxyl group is substituted by an amino group or a fluorine atom, said another L-glucose being linked to sulforhodamine.

12. The compound for use according to claim 11, wherein the L-glucose linked to sulforhodamine is 2-amino-2-deoxy-L-glucose linked to sulforhodamine 101 or sulforhodamine B at the 2-position by way of a sulfonamide bond.

13. The compound for use according to claim 8, wherein the L-glucose linked to a 7-nitrobenz-2-oxa-1,3-diazole group or a 7-(N,N-dimethylaminosulfonyl)benz-2-oxa-1,3-diazole group is 6-[¹⁸F]-2-[N-(7-nitrobenz-2-oxa-1,3-diazole-4-yl)amino]-2-deoxy-L-glucose or 4-[¹⁸F]-2-[N-(7-nitrobenz-2-oxa-1,3-diazole-4-yl)amino]-2-deoxy-L-glucose.

14. The compound for use according to claim 13 for use in an *in vivo* diagnosing method for confirming the site of cancer cells with a whole body imaging using PET.

## Patentansprüche

1. Ein Verfahren zum Nachweis von Krebszellen *in vitro,* umfassend die folgenden Schritte:
(a) einen Schritt, in dem eine Zusammensetzung enthaltend eine L-Glucose oder eine L-Glucose, in der eine Hydroxylgruppe durch eine Aminogruppe oder ein Fluoratom ersetzt wurde, wobei diese L-Glucose über ein Amin oder über einen Spacer repräsentiert durch die Formel -NH-(CH₂)ₙ-NH-, worin n 2 bis 20 ist, verlinkt ist mit einer 7-Nitrobenz-2-oxa-1,3-diazolgruppe oder einer 7-(N,N-Dimethylaminosulfonyl)benz-2-oxa-1,3-diazolgruppe, oder eine Salzform davon, *in vitro* in Kontakt gebracht wird mit einer Targetzelle,
(b) einen Schritt, in dem die in der Targetzelle vorhandene, mit der 7-Nitrobenz-2-oxa-1,3-diazolgruppe oder 7-(N,N-Dimethylaminosulfonyl)benz-2-oxa-1,3-diazolgruppe verlinkte L-Glucose detektiert wird durch die Detektion von Fluoreszenz, und
(c) einen Schrit, in dem eine Krebszelle nachgewiesen wird, basierend auf einem Ansteigen der Fluoreszenzintensität in der Targetzelle.

2. Das Nachweisverfahren gemäß Anspruch 1, wobei die mit der 7-Nitrobenz-2-oxa-1,3-diazolgruppe oder 7-(N,N-Dimethylaminosulfonyl)benz-2-oxa-1,3-diazolgruppe verlinkte L-Glucose erhältlich ist durch Verlinken einer 7-Nitrobenz-2-oxa-1,3-diazolgruppe oder einer 7-(N,N-Dimethylaminosulfonyl)benz-2-oxa-1,3-diazolgruppe mit der 2-Position, 4-Position oder 6-Position der L-Glucose.

3. Das Nachweisverfahren gemäß Anspruch 1 oder 2, wobei die mit der 7-Nitrobenz-2-oxa-1,3-diazolgruppe oder 7-(N,N-Dimethylaminosulfonyl)benz-2-oxa-1,3-diazolgruppe verlinkte L-Glucose 2-[N-(7-Nitrobenz-2-oxa-1,3-diazole-4-yl)amino]-2-deoxy-L-glucose oder 2-Deoxy-2-[N-7-(N´,N´-dimethylaminosulfonyl)benz-2-oxa-1,3-diazole-4-yl)amino]-L-glucose ist.

4. Das Nachweisverfahren gemäß irgendeinem der Ansprüche 1 bis 3, wobei die Detektion im Schritt (b) durch einer Bildaufnahme der Targetzelle erfolgt.

5. Das Nachweisverfahren gemäß irgendeinem der Ansprüche 1 bis 4, wobei die Zusammensetzung im Schritt (a) des Weiteren eine anderer L-Glucose oder eine andere L-Glucose, in der eine Hydroxylgruppe durch eine Aminogruppe oder ein Fluoratom ersetzt wurde, enthält, wobei diese andere L-Glucose mit Sulforhodamin verlinkt ist, wobei die L-Glucose aus der Zusammensetzung gemäß Schritt (a) und diese andere L-Glucose unterschiedlich in Zellen aufgenommen werden und bei unterschiedlichen Wellenlängen detektiert werden, wodurch ein Rauschen durch Aufnahme aufgrund von Zellmembranschäden wegen Entzündung oder physischer Verletzungen und aufgrund von unspezifischer Aufnahme von Macrophagen ausgeschlossen wird.

6. Das Nachweisverfahren gemäß Anspruch 5, wobei die Zusammensetzung 2-[N-(7-Nitrobenz-2-oxa-1,3-diazole-4-yl)amino]-2-deoxy-L-glucose und 2-Amino-2-deoxy-L-glucose, welche über eine Sulfonamidbindung an der 2-Position mit Sulforhodamin 101 oder Sulforhodamin B verlinkt ist, enthält.

7. Das Nachweisverfahren gemäß Anspruch 5 oder Anspruch 6, wobei die Detektion im Schritt (b) durchgeführt wird mit Hilfe einer temporalen Veränderung im fluoreszenten Farbton der abgebildeten Zelle als einem Index.

8. Eine Verbindung zur Verwendung in einem *in vivo*-Diagnoseverfahren zum Nachweis von Krebszellen, wobei diese Verbindung eine L-Glucose oder eine L-Glucose, in der eine Hydroxylgruppe durch eine Aminogruppe oder ein Fluoratom ersetzt wurde, wobei diese L-Glucose über ein Amin oder über einen Spacer repräsentiert durch die Formel -NH-(CH₂)ₙ-NH-, worin n 2 bis 20 ist, verlinkt ist mit einer 7-Nitrobenz-2-oxa-1,3-diazolgruppe oder einer 7-(N,N-Dimethylaminosulfonyl)benz-2-oxa-1,3-diazolgruppe, oder eine Salzform davon ist, wobei das Diagnoseverfahren die folgenden Schritte umfasst:
(1) lokales oder systemisches Verabreichen dieser Verbindung an einen lebenden Körper,
(2) Durchführen eines bildgebenden Verfahrens, und
(3) Evaluieren der Aufnahme dieser Verbindung in eine Zelle durch Detektion eines Anstiegs der Fuoreszenzintensität, und so den Nachweis von Krebszellen führen.

9. Die Verbindung zur Verwendung gemäß Anspruch 8, wobei die mit der 7-Nitrobenz-2-oxa-1,3-diazolgruppe oder 7-(N,N-Dimethylaminosulfonyl)benz-2-oxa-1,3-diazolgruppe verlinkte L-Glucose erhältlich ist durch Verlinken einer 7-Nitrobenz-2-oxa-1,3-diazolgruppe oder einer 7-(N,N-Dimethylaminosulfonyl)benz-2-oxa-1,3-diazolgruppe mit der 2-Position, 4-Position oder 6-Position der L-Glucose.

10. Die Verbindung zur Verwendung gemäß Anspruch 8 oder 9, wobei die mit der 7-Nitrobenz-2-oxa-1,3-diazolgruppe oder 7-(N,N-Dimethylaminosulfonyl)benz-2-oxa-1,3-diazolgruppe verlinkte L-Glucose 2-[N-(7-Nitrobenz-2-oxa-1,3-diazole-4-yl)amino]-2-deoxy-L-glucose oder 2-Deoxy-2-[N-7-(N´,N´-dimethylaminosulfonyl)benz-2-oxa-1,3-diazole-4-yl)amino]-L-glucose ist.

11. Die Verbindung zur Verwendung gemäß irgendeinem der Ansprüche 8 bis 10, wobei das bildgebende Reagenz des Weiteren eine anderer L-Glucose oder eine andere L-Glucose, in der eine Hydroxylgruppe durch eine Aminogruppe oder ein Fluoratom ersetzt wurde, enthält, wobei diese andere L-Glucose mit Sulforhodamin verlinkt ist.

12. Die Verbindung zur Verwendung gemäß Anspruch 11, wobei die mit Sulforhodamin verlinkte L-Glucose 2-Amino-2-deoxy-L-glucose ist, welche über eine Sulfonamidbindung an der 2-Position mit Sulforhodamin 101 oder Sulforhodamin B verlinkt ist.

13. Die Verbindung zur Verwendung gemäß Anspruch 8, wobei die mit der 7-Nitrobenz-2-oxa-1,3-diazolgruppe oder 7-(N,N-Dimethylaminosulfonyl)benz-2-oxa-1,3-diazolgruppe verlinkte L-Glucose 6-[¹⁸F]-2-[N-(7-Nitrobenz-2-oxa-1,3-diazole-4-yl)amino]-2-deoxy-L-glucose oder 4-[¹⁸F]-2-[N-(7-Nitrobenz-2-oxa-1,3-diazole-4-yl)amino]-2-deoxy-L-glucose ist.

14. Die Verbindung zur Verwendung gemäß Anspruch 13 zur Verwendung in einem *in vivo*-Diagnoseverfahren zur Bestätigung der Lage von Krebszellen mittels eines Ganzkörperbildes durch PET.

## Revendications

1. Méthode de détection de cellules cancéreuses *in vitro,* comprenant les étapes suivantes :
(a) une étape consistant à amener une composition contenant un L-glucose ou un L-glucose dans lequel un groupe hydroxyle est substitué par un groupe amino ou un atome de fluor, ledit L-glucose étant lié à un groupe 7-nitrobenz-2-oxa-1,3-diazole ou un groupe 7-(N,N-diméthylaminosulfonyl)benz-2-oxa-1,3-diazole par l'intermédiaire d'une amine ou par l'intermédiaire d'un espaceur représenté par la formule -NH-(CH₂)ₙ-NH-, formule dans laquelle n vaut 2 à 20, ou une forme de sel de celui-ci, en contact avec une cellule cible *in vitro,*
(b) une étape consistant à détecter le L-glucose lié au groupe 7-nitrobenz-2-oxa-1,3-diazole ou au groupe 7-(N,N-diméthylaminosulfonyl)benz-2-oxa-1,3-diazole présent dans la cellule cible par détection de fluorescence, et
(c) une étape consistant à détecter une cellule cancéreuse sur la base d'une augmentation de l'intensité de fluorescence dans la cellule cible.

2. Méthode de détection selon la revendication 1, dans laquelle le L-glucose lié à un groupe 7-nitrobenz-2-oxa-1,3-diazole ou un groupe 7-(N,N-diméthylaminosulfonyl)benz-2-oxa-1,3-diazole est obtenu par liaison d'un groupe 7-nitrobenz-2-oxa-1,3-diazole ou d'un groupe 7-(N,N-diméthylaminosulfonyl)benz-2-oxa-1,3-diazole à la position 2, à la position 4 ou à la position 6 du L-glucose.

3. Méthode de détection selon la revendication 1 ou 2, dans laquelle le L-glucose lié à un groupe 7-nitrobenz-2-oxa-1,3-diazole ou un groupe 7-(N,N-diméthylaminosulfonyl)benz-2-oxa-1,3-diazole est le 2-[N-(7-nitrobenz-2-oxa-1,3-diazole-4-yl)amino]-2-désoxy-L-glucose ou le 2-désoxy-2-[N-7-(N',N'-diméthylaminosulfonyl)benz-2-oxa-1,3-diazole-4-yl)amino]-L-glucose.

4. Méthode de détection selon l'une quelconque des revendications 1 à 3, dans laquelle la détection à l'étape (b) est conduite par imagerie de la cellule cible.

5. Méthode de détection selon l'une quelconque des revendications 1 à 4, dans laquelle la composition à l'étape (a) contient en outre un autre L-glucose ou un autre L-glucose dans lequel un groupe hydroxyle est substitué par un groupe amino ou un atome de fluor, ledit autre L-glucose étant lié à une sulforhodamine, le L-glucose de la composition de l'étape (a) et ledit autre L-glucose étant absorbés différemment dans les cellules et détectés à des longueurs d'onde différentes, ce qui permet d'exclure les bruits de l'absorption attribuable aux dommages de la membrane cellulaire en raison d'une inflammation ou d'une blessure physique, et de l'absorption non spécifique de macrophage.

6. Méthode de détection selon la revendication 5, dans laquelle la composition contient du 2-[N-(7-nitrobenz-2-oxa-1,3-diazole-4-yl)amino]-2-désoxy-L-glucose et du 2-amino-2-désoxy-L-glucose lié à la sulforhodamine 101 ou à la sulforhodamine B à la position 2 au moyen d'une liaison sulfonamide.

7. Méthode de détection selon la revendication 5 ou la revendication 6, dans laquelle la détection à l'étape (b) est conduite à l'aide d'une modification temporelle de la nuance de couleur en fluorescence de la cellule imagée en tant qu'indice.

8. Composé pour une utilisation dans une méthode de diagnostic *in vivo* pour détecter des cellules cancéreuses, ledit composé étant un L-glucose ou un L-glucose dans lequel un groupe hydroxyle est substitué par un groupe amino ou un atome de fluor, ledit L-glucose étant lié à un groupe 7-nitrobenz-2-oxa-1,3-diazole ou un groupe 7-(N,N-diméthylaminosulfonyl)benz-2-oxa-1,3-diazole par l'intermédiaire d'une amine ou par l'intermédiaire d'un espaceur représenté par la formule -NH-(CH₂)ₙ-NH-, formule dans laquelle n vaut 2 à 20, ou une forme de sel de celui-ci, ladite méthode de diagnostic comprenant les étapes consistant à :
(1) administrer ledit composé à un corps vivant de manière locale ou de manière systémique,
(2) effectuer une imagerie, et
(3) évaluer l'absorption dudit composé dans une cellule par détection d'une augmentation de l'intensité de fluorescence, et ainsi détecter des cellules cancéreuses.

9. Composé pour une utilisation selon la revendication 8, dans lequel le L-glucose lié à un groupe 7-nitrobenz-2-oxa-1,3-diazole ou un groupe 7-(N,N-diméthylaminosulfonyl)benz-2-oxa-1,3-diazole est obtenu par liaison d'un groupe 7-nitrobenz-2-oxa-1,3-diazole ou d'un groupe 7-(N,N-diméthylaminosulfonyl)benz-2-oxa-1,3-diazole à la position 2, à la position 4 ou à la position 6 du L-glucose.

10. Composé pour une utilisation selon la revendication 8 ou 9, dans lequel le L-glucose lié au groupe 7-nitrobenz-2-oxa-1,3-diazole ou au groupe 7-(N,N-diméthylaminosulfonyl)benz-2-oxa-1,3-diazole est le 2-[N-(7-nitrobenz-2-oxa-1,3-diazole-4-yl)amino]-2-désoxy-L-glucose ou le 2-désoxy-2-[N-7-(N',N'-diméthylaminosulfonyl)benz-2-oxa-1,3-diazole-4-yl)amino]-L-glucose.

11. Composé pour une utilisation selon l'une quelconque des revendications 8 à 10, dans lequel l'agent d'imagerie contient en outre un autre L-glucose ou un autre L-glucose dans lequel un groupe hydroxyle est substitué par un groupe amino ou un atome de fluor, ledit autre L-glucose étant lié à une sulforhodamine.

12. Composé pour une utilisation selon la revendication 11, dans lequel le L-glucose lié à une sulforhodamine est le 2-amino-2-désoxy-L-glucose lié à la sulforhodamine 101 ou à la sulforhodamine B à la position 2 au moyen d'une liaison sulfonamide.

13. Composé pour une utilisation selon la revendication 8, dans lequel le L-glucose lié à un groupe 7-nitrobenz-2-oxa-1,3-diazole ou un groupe 7-(N,N-diméthylaminosulfonyl)benz-2-oxa-1,3-diazole est le 6-[¹⁸F]-2-[N-(7-nitrobenz-2-oxa-1,3-diazole-4-yl)amino]-2-désoxy-L-glucose ou le 4-[¹⁸F]-2-[N-(7-nitrobenz-2-oxa-1,3-diazole-4-yl)amino]-2-désoxy-L-glucose.

14. Composé pour une utilisation selon la revendication 13 pour une utilisation dans une méthode de diagnostic *in vivo* pour confirmer l'emplacement de cellules cancéreuses avec une imagerie du corps entier à l'aide de TEP.
